# FASCICULE DE BREVET EUROPEEN

(11) **EP 2 331 710 B1**
(45) Date de publication et mention de la délivrance du brevet: **22.05.2013**
(21) Numéro de dépôt: 09740491.7
(22) Date de dépôt: 25.08.2009
(51) Int. Cl.: C12Q 1/68

(54) **MARQUEURS GENETIQUES DE LA COLORATION DE LA VIANDE**
GENETISCHE MARKER FÜR FLEISCHFARBE
GENETIC MARKERS FOR MEAT COLOUR

(30) Priorité: 25.08.2008 EP 08305500
(43) Date de publication de la demande: 15.06.2011
(73) Titulaire: Institut National de la Recherche Agronomique, 75338 Paris (FR)
(72) Inventeur: DUVAL, Elisabeth, F-37270 Montlouis-sur-Loire (FR); NADAF, Javad, Mashhad (IR); BERRI, Cécile, F-37230 Fondettes (FR); DUCLOS, Michel, F-37210 Vernou-sur-Brenne (FR); PITEL, Frédérique, F-31460 Beauville (FR)
(74) Mandataire: Cabinet Plasseraud
(86) Numéro de dépôt international: PCT/FR2009/051627
(87) Numéro de publication internationale: WO 2010/023407

(56) Documents cités:
- WO-A-2005/040400
- WO-A-2008/031846
- NADAF JAVAD ET AL: "Identification of QTL controlling meat quality traits in an F-2 cross between two chicken lines selected for either low or high growth rate" BMC GENOMICS, vol. 8, juin 2007 (2007-06), page Article No.: 155, XP002509599 ISSN: 1471-2164
- GONG XIAOMING ET AL: "Cooperation between MEF2 and PPAR gamma in human intestinal beta,beta-carotene 15,15 '-monooxygenase gene expression" BMC MOLECULAR BIOLOGY, vol. 7, février 2006 (2006-02), XP002509600 ISSN: 1471-2199

## Description

La présente divulgation concerne la différenciation et la sélection d'animaux dans une population, en particulier des poulets, en fonction de la coloration de leur viande et en particulier présentant une viande la plus ou la moins colorée et l'obtention d'animaux présentant une viande plus colorée. Plus précisément la présente divulgation concerne un procédé de sélection d'animaux dans une population présentant une viande la plus ou la moins colorée et un procédé d'obtention d'animaux présentant une viande plus colorée par la mise en évidence de marqueurs génétiques de la coloration de la viande.

L'apparence des produits alimentaires joue un rôle important dans la décision du consommateur entre acheter et ne pas acheter un aliment. Des études ont montré que notamment pour les viandes blanches (volailles, canard, porc, lapin, veau) le consommateur préfère soit les viandes les plus colorées c'est-à-dire celles présentant une couleur jaune, et dans une moindre mesure rouge, la plus importante, soit les viandes les moins colorées. Le choix d'une viande blanche plus ou moins colorée par le consommateur est fonction notamment du pays et du type de viande : ainsi par exemple, les études de consommateurs ont pu noter que le consommateur français recherche une viande de volaille la plus colorée et une viande de veau la moins colorée. Le consommateur Italien recherche quant à lui une viande de veau la plus colorée.

En outre, ces dernières années, les animaux à viande blanche à destination de l'alimentation humaine, et en particulier les poulets, ont été sélectionnés de manière intensive sur la base de leur vitesse de croissance, ce qui a permis de réduire l'âge de mise sur le marché de l'animal.

Ces efforts de sélection ont permis d'améliorer la composition corporelle (c'est-à-dire augmenter la taille des filets et diminuer le taux de matière grasse de la viande).

Toutefois, ces améliorations ont également conduit indirectement à des effets nuisibles sur la qualité de la viande (c'est-à-dire, la couleur de la viande, le pH et la capacité de rétention d'eau).

L'importance de ces effets nuisibles a augmenté suite à la mise au point de produits alimentaires obtenus par une transformation plus poussée de la viande, en particulier les volailles.

Il existe donc aujourd'hui un besoin
1. d'identifier et de sélectionner les animaux parmi une population ayant la viande la plus ou la moins colorée, et ce si possible sans les sacrifier afin de pouvoir effectuer des croisements avec lesdits animaux ;
2. d'augmenter ou de diminuer la couleur de la viande d'animaux, en particulier à viande blanche, en fonction des attentes du consommateur ;
3. d'améliorer la qualité de la viande, notamment des animaux à viande blanche, en particulier des poulets, issus de la sélection intensive, et en particulier d'augmenter la couleur de la viande (c'est-à-dire augmenter la couleur jaune, et dans une moindre mesure rouge,) tout en conservant les améliorations de la composition corporelle et la bonne vitesse de croissance des animaux.

La sélection classique, par mesure directe de la couleur, reste difficile à mettre en oeuvre, car elle implique une sélection sur collatéraux, qui reste coûteuse et moins efficace.

La présente divulgation répond au problème de différenciation, sélection et d'obtention des volailles, présentant une viande plus ou moins colorée grâce à la mise en évidence du gène BCMO1 codant pour la beta-carotène monooxygénase 1 comme marqueur génétique de la coloration de la viande et de l'association entre la sous-expression du gène BCMO1 et une viande plus colorée.

Ainsi, la présente divulgation concerne dans un premier aspect un procédé pour différencier dans une population des volailles les animaux en fonction de la coloration de leur viande, caractérisé en ce qu'il comprend, pour un animal de ladite population les étapes suivantes:
a. Identification d'au moins une mutation associée à la sous-expression ou à la surexpression du gène BCMO1, dans un échantillon biologique dudit animal ; et/ou
b. Analyse de l'expression du gène BCMO1 dans un échantillon biologique dudit animal ;
où une mutation associée à la sous-expression du gène BCMO1, une mutation associée à la diminution de l'activité de la protéine BCMO1, une sous-expression du gène BCMO1, ou une diminution de l'activité de la protéine BCMO1 permet de différencier les animaux présentant la viande la plus colorée dans une population et inversement, une mutation associée à la sur-expression du gène BCMO1, une mutation associée à l'augmentation de l'activité de la protéine BCMO1, une sur-expression du gène BCMO1, ou une augmentation de l'activité de la protéine BCMO1 permet de différencier les animaux présentant la viande la moins colorée dans une population.

Selon un autre aspect, la présente divulgation concerne un procédé de sélection d'animaux présentant une viande la plus colorée dans une population, caractérisé en ce qu'il comprend les étapes suivantes :
a. Identification d'au moins une mutation associée à la sous-expression du gène BCMO1 dans un échantillon biologique dudit animal ; et/ou
b. Analyse de l'expression du gène BCMO1 dans un échantillon biologique dudit animal ; et
c. Sélection des animaux dont l'échantillon biologique présente une mutation associée à la sous-expression du gène BCMO1 et/ou une mutation associée à la diminution de l'activité de la protéine BCMO1, et/ou une sous-expression du gène BCMO1.

De manière préférée selon la présente divulgation, les animaux sont des volailles, de préférence des poules (communément appelés poulets).

La couleur de la viande peut être mesurée par toute technique connue de l'Homme du métier, par exemple en utilisant un spectrocolorimètre avec le système CIE L*a*b*, où L* correspond à la luminosité, a* correspond à la teinte rouge et b* à la teinte jaune. Des valeurs plus importantes des valeurs de L*, a* et b* correspondent à des viandes respectivement plus pâles, plus rouges et plus jaunes. De ce fait, lorsqu'il est recherché les viandes les plus colorées, les valeurs de a* et b* les plus importantes et la valeur de L* la moins importante sont recherchées. A l'inverse, lorsqu'il est recherché les viandes les moins colorées, les valeurs de a* et b* les moins importantes et la valeur de L* la plus importante sont recherchées.

Les valeurs de L*, a* et b* des témoins sont susceptibles d'être différentes selon les espèces animales et l'homme du métier saura déterminer facilement pour chaque espèce les valeurs de L*, a* et b* au-dessus ou en-dessous desquelles il souhaite différencier ou sélectionner les animaux, grâce à la littérature ou tout simplement en testant le témoin négatif (chair pas assez colorée ou trop colorée) avec un spectrocolorimètre avec le système CIE L*a*b*.

Le tableau ci-après présente des exemples de valeurs de L*a*b* d'animaux témoins :

**Tableau 1 : Couleur de la viande : paramètres L*, a*, b***

| | L* | a* | b* | Références |
|---|---|---|---|---|
| Filet de poulet âgé de 9 semaines des lignées expérimentales à croissance lente (CL) ou rapide (CR) | CL= 45,6 | CL=1,6 | CL=13,3 | Nadaf et al., 2007 |
| | CR=48,3 | CR=-0,2 | CR=9,4 | |
| Filet de poulet âgé de 9 semaines des lignées expérimentales « maigre » (M) ou « grasse » (G) | M=44,9 | M=-0,3 | M=9,3 | Sibut et al., 2008 |
| | G=47,4 | G=-1,0 | G=8,3 | |
| Filet de poulet âgé de 6 semaines d'une lignée expérimentale sélectionnée (S) sur la conformation et sa lignée témoin (T) | S=49,8 | S=0,2 | S=10,4 | Berri et al., 2001 |
| | T=48,4 | T=0,6 | T=10,9 | |
| Filet de poulet âgé de 6 semaines d'une lignée commerciale de poulet lourd (L) et sa lignée témoin (T) | L=49,7 | L=-1,0 | L=7,5 | Berri et al., 2001 |
| | T=48,1 | T=0,3 | T=9,4 | |
| Filet de poulet âgé de 6 semaines d'une lignée commerciale de poulet lourd | 54,9 | -0,8 | 11,8 | Le Bihan-Duval et al., 2008 |
| Filet de poulet standard de 6 semaines (S) et de poulet label de 12 semaines (L) | S=49,8 | S=-0,6 | S=8,4 | Gigaud et al., 2007 |
| | L=51,2 | L=-1,0 | L=10,4 | |
| Filet de canard de Barbarie âgé de 8 semaines | 45,7 | 8,3 | 12,4 | Baéza et al., 1998a |
| Filet de canette de Barbarie âgée de 8 semaines | 42,4 | 8,4 | 9,7 | idem |
| Filet de canard de Barbarie âgé de 10 semaines | 39,7 | 12,9 | 12,4 | idem |
| Filet de canette de Barbarie âgée | 37,0 | 13,3 | 11,0 | idem |
| de 10 semaines (âge usuel d'abattage) | | | | |
| Filet de canard de Barbarie âgé de 12 semaines (âge usuel d'abattage) | 35,7 | 12,5 | 9,9 | idem |
| Filet de canette de Barbarie âgée de 12 semaines | 36,1 | 14,0 | 10,7 | idem |
| Filet de canard de Barbarie âgé de 15 semaines | 35,4 | 16,1 | 12,4 | idem |
| Filet de canette de Barbarie âgée de 15 semaines | 37,0 | 16,2 | 13,6 | idem |
| Filet d'oie landaise non gavée âgée de 24 semaines | 36,3 | 12,3 | 10,3 | Baéza et al., 1998b |
| Filet d'oie landaise gavée âgée de 26 semaines | 39,7 | 13,0 | 11,5 | idem |
| Filet d'oie landaise non gavée âgée de 14 semaines | 43,9 | 11,1 | 12,4 | idem |
| Filet d'oie landaise gavée âgée de 16 semaines | 43,5 | 11,8 | 13,6 | idem |
| Filet de canard mulard âgé de 8 semaines | 41,7 | 11,6 | 12,9 | Baéza et al., 2000 |
| Filet de canette mulard âgée de 8 semaines | 41,1 | 11,7 | 12,6 | idem |
| Filet de canard mulard âgé de 10 semaines (âge usuel d'abattage) | 40,0 | 12,0 | 12,7 | idem |
| Filet de canette mulard âgée de 10 semaines (âge d'abattage) | 41,2 | 12,0 | 12,5 | idem |
| Filet de canard mulard âgé de 12 semaines | 37,4 | 13,2 | 12,4 | idem |
| Filet de canette mulard âgée de 12 semaines | 39,7 | 11,8 | 12,4 | idem |
| Filet de canard Pékin gavé et âgé de 14 semaines | 41,5 | 13,8 | 13,6 | Chartrin et al., 2006 |
| Filet de canard Pékin non gavé et âgé de 14 semaines | 30,8 | 13,7 | 8,2 | idem |
| Filet de canard mulard gavé et âgé de 14 semaines | 41,2 | 14,2 | 14,3 | idem |
| Filet de canard mulard non gavé et âgé de 14 semaines | 31,9 | 13,8 | 8,9 | idem |
| Filet de canard hinny gavé et âgé de 14 semaines | 42,9 | 14,1 | 15,0 | Chartrin et al., 2006 |
| Filet de canard hinny non gavé et âgé de 14 semaines | 32,2 | 13,9 | 9,2 | idem |
| Filet de canard de Barbarie gavé et âgé de 14 semaines | 43,6 | 12,8 | 14,7 | idem |
| Filet de canard de Barbarie non gavé et âgé de 14 semaines | 35,0 | 13,6 | 11,4 | idem |
| Filet de dinde BUT de 16 | 51,0 | 3,7 | 5,2 | Fernandez et |
| semaines (pH 20 min = 5,90) | | | | al., 2002 |
| Filet de dinde BUT de 16 semaines (pH 20 min = 6,24) | 49,9 | 3,2 | 5,1 | idem |
| Filet de dinde BUT de 16 semaines (pH 20 min = 6,55) | 49,5 | 2,8 | 5,4 | idem |
| Longissimus dorsi, porc de 110 kg | 54,2 | 5,5 | 5,0 | Lebret et al., 2007 |
| Biceps femoris, porc de 110 kg | 51,2 | 10,7 | 6,2 | idem |
| Semimembranosus, porc de 110 kg | 53,0 | 9,0 | 6,4 | idem |
| Longissimus thoracis et lumborum, porc de 107 kg | 50,16 | 6,36 | 14,45 | Apple et al., 2007 |
| Longissimus dorsi, porc de 100-110 kg | 52,6 | 7,4 | 4,0 | Skrlep et Candek-Potokar, 2007 |
| Longissimus dorsi, boeuf charolais X frisonne de 23 mois | 42,7 | 19,2 | 14,5 | Moloney et al., 2008 |
| Longissiumus dorsi, boeuf frison danois de 550 kg | 32,6 | 20,6 | 9,5 | Vestegaard et al., 2007 |
| Psoas major, boeuf de 340-390 kg | 46,3 | 33,4 | 25,7 | Seyfert et al., 2006 |
| Longissimus lumborum, boeuf de 340-390 kg | 41,3 | 34,4 | 27,1 | idem |
| Semimembranosus, boeuf de 340-390 kg | 40,7 | 33,6 | 27,4 | Idem |
| Semimembranosus profond, boeuf de 340-390 kg | 51,9 | 35,1 | 29,5 | Idem |
| Semitendinosus, boeuf de 340-390 kg | 47,6 | 32,2 | 27,1 | idem |

Ainsi par exemple pour le poulet, le témoin est une viande avec une valeur du b* voisine de 10, une valeur du a* voisine de 0 et une valeur du L* voisine de 49.

De ce fait, un procédé de sélection de poulets présentant une viande la plus colorée dans une population correspondra à une sélection de poulets présentant une viande qui, mesurée à l'aide d'un spectrocolorimètre avec le système CIE L*a*b*, aura une valeur du b* supérieure à 10, une valeur du a* supérieure à 0 et une valeur du L* inférieure à 49.

A l'inverse, un procédé de sélection de poulets présentant une viande la moins colorée dans une population correspondra à une sélection de poulets présentant une viande qui, mesurée à l'aide d'un spectrocolorimètre avec le système CIE L*a*b*, aura une valeur du b* inférieure à 10, une valeur du a* inférieure à 0 et une valeur du L* supérieure à 49.

Par échantillon biologique, on entend selon la présente divulgation des tissus solides tels que par exemple un morceau de viande, un morceau de peau, des fluides ou excrétions tels que par exemple du sang, du sérum, du plasma. De préférence, ledit échantillon biologique est un échantillon de fluide et de manière encore plus préférée un échantillon de sang. De préférence ledit échantillon biologique comprend des cellules.

Par gène BCMO1, on entend selon la présente divulgation le gène codant pour la Béta-carotène 15,15'-monooxygénase, anciennement BCDO1 (Béta, béta-carotène dioxygénase 1) de séquence SEQ ID N° 1 (Numéro d'accession GO: 0003834 ; GenBank : AJ271386). Le gène BCMO1 est présent sur la séquence du génome de la poule sur l'assemblage du chromosome 11 entre les bases 17 085 000 et 17 105 000 (assemblage version 2.1, Ensembl Genome Browser, http://www.ensembl.org/index.html). Le transcrit a une taille de 3 046 paires de bases (pb), pour une protéine de 526 acides aminés (1). Les informations disponibles sur la fonction du gène indiquent que BCMO1 code pour l'enzyme β, β-carotène 15,15' - monooxygénase.

Par mutation associée à la sous-expression du gène BCMO1, on entend de préférence selon la présente divulgation un polymorphisme nucléotidique (ou SNP, en anglais Single Nucleotide Polymorphism). De préférence, ladite au moins une mutation a la même localisation chromosomale que le gène BCMO1.

De manière encore préférée ladite au moins une mutation est située dans la région promotrice du gène BCMO1.

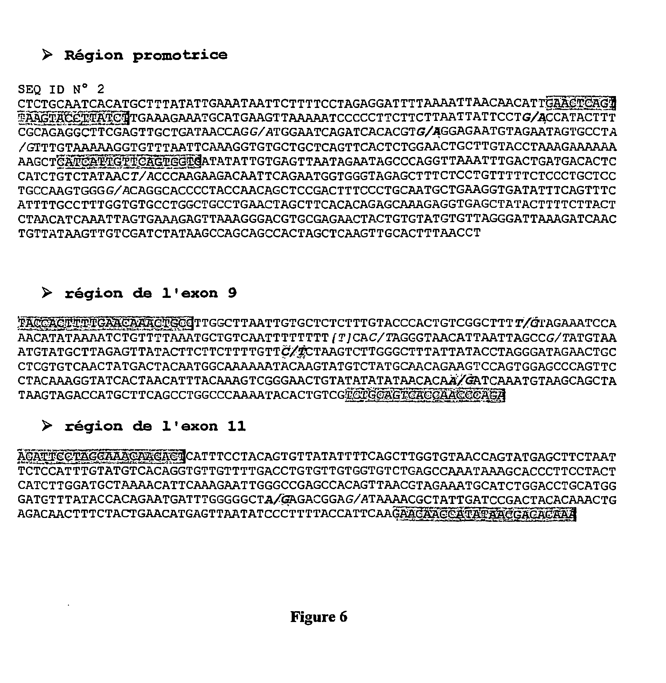

De manière encore plus préférée ladite au moins une mutation est la mutation A/G à la position 139 de SEQ ID N°2 et/ou la mutation A/G à la position 197 de SEQ ID N°2 (Figure 6).

Par mutation associée à une diminution de l'activité de la protéine BCMO1, on entend selon la présente invention une mutation, de préférence l'insertion d'un codon stop ou une substitution, entraînant l'expression d'une protéine BCMO1 non-active ou moins active. De préférence une telle mutation n'est pas localisée dans le promoteur.

L'homme du métier appréciera immédiatement que les informations présentées ci-avant concernant le gène BCMO1 de la poule sont facilement associées ou corrélées à une mutation similaire à une localisation correspondante pour le gène BCMO1 d'autres espèces.

L'activité de l'enzyme béta-carotène 15,15'-dioxygénase (BCMO1) est déterminée dans un échantillon biologique par tout moyen connu de l'Homme du métier, par exemple grâce à une méthode in vitro de mesure de l'efficacité de conversion du tout-trans béta-carotène en rétinal en présence de fractions protéiques tissulaires purifiées. Ces fractions protéiques sont préparées par la combinaison de centrifugations, permettant d'obtenir un surnageant exempt de mitochondries, et de lavages sur colonnes afin d'éliminer des composés pouvant potentiellement interférer dans le dosage. La réaction enzymatique est réalisée au cours d'une incubation en conditions contrôlées (teneur en protéines tissulaires, concentrations de substrat, de cofacteurs, d'agents antioxydants et solubilisants, température, durée). Le tout-trans béta-carotène résiduel et le rétinal néoformé à l'issue de l'incubation sont quantifiés par chromatographie liquide ultra-haute pression. L'activité enzymatique est exprimée en nombre de moles de tout-trans béta-carotène biotransformé ou en nombre de moles de rétinal produites par minute et par milligramme de protéines tissulaires.

Selon un aspect de la présente divulgation, le procédé comprend une étape préalable à l'étape a. de transformation des cellules dudit animal pour qu'elles présentent une mutation associée à la sous-expression du gène BCMO1 et/ou une sous-expression du gène BCMO1.

Des techniques pour détecter une telle mutation pouvant être de manière préférée un SNP, incluent par exemple les techniques de polymorphisme de longueurs des fragments de restriction (RFLP: Restriction Fragment Length Polymorphism) ou de conformation en simple brin (SSCP: Single Strand Conformation Polymorphism), la technique de fusion haute-résolution (HRM: High Resolution Melting), les techniques de séquençage d'ADN, de résistance à l'exonucléase, de micro-séquençage, la technique de ligature d'oligonucléotides, les techniques d'hybridation, comme par exemple la technique d'hybridation allèle spécifique dynamique, les puces à ADN, la technique d'hybridation allèle spécifique avec simple ou double sondes marquées associé à une PCR ou un phare moléculaire ou autres.

L'analyse de l'expression du gène BCMO1 peut être effectuée par tout type de techniques bien connues de l'homme du métier pour détecter l'expression d'un acide nucléique transcrit ou une protéine traduite.

De manière préférée, l'expression du gène BCMO1 est analysée par la détection de l'expression des ARNm transcrits du gène BCMO1 ou des précurseurs des ARNm, tel que les ARN naissants, dudit gène. De telles analyses peuvent être effectuées par la préparation d'ARNm/ADNc à partir de cellules dans un échantillon biologique d'un animal, et l'hybridation dudit ARNm/ADNc avec un polynucléotide de référence.

L'ARNm/ADNc préparé peut être utilisé dans des méthodes d'hybridation ou d'amplification qui incluent, mais ne sont pas limitées à, des analyses de type Southern ou Northern, PCR telles que la PCR quantitative, et des puces à acides nucléiques.

De manière avantageuse, l'analyse de l'expression de la quantité d'ARNm transcrits à partir du gène BCMO1 implique un procédé d'amplification d'acide nucléique, par exemple par RT-PCR (tel que décrit dans le brevet US 4,683,202), réaction de ligature en chaîne (BARANY, Proc, Natl. Acad. Sci USA vol.88 p : 189-193, 1991), système d'amplification transcriptionel (KWOH et al., 1989, Proc Natl. Acad. Sci USA vol.86 p :1173-1177, 1989), Replicase Q-Beta (LIZARDI et al. Biol. Technology, Vol.6, p :1197, 1988) ou tout autre type de procédé d'amplification d'acides nucléiques, suivie par la détection des molécules amplifiées, en utilisant des techniques bien connues de l'Homme du métier. Ces schémas de détection sont particulièrement utiles pour la détection d'acides nucléiques lorsque ceux-ci sont présents en faibles quantités. Tels que décrits ici, les amorces d'amplification sont définies comme étant une paire d'acides nucléiques pouvant s'hybrider à la région 5' ou 3' d'un gène (brin plus ou brin moins, respectivement ou vice versa) et contenant une région courte au milieu. De manière générale, les amorces d'hybridation contiennent 10 à 30 nucléotides de longueur et encadrent une région d'environ 50 à environ 200 nucléotides de longueur.

Sous conditions appropriées, et avec les réactifs appropriés, de telles amorces permettent l'amplification d'un acide nucléique comprenant la séquence nucléotidique encadrée par les amorces.

Selon un autre aspect préféré de la divulgation, l'expression du gène BCMO1 peut être déterminée par la quantification de la protéine BCMO1. Une telle analyse peut être effectuée en utilisant un anticorps (par exemple un anticorps radio-marqué, marqué par un chromophore, marqué par un fluorophore ou marqué par une enzyme), un dérivé d'anticorps (par exemple un conjugué d'anticorps avec un substrat ou avec la protéine ou le ligand d'une protéine d'un couple protéine/ligand (par exemple le couple biotine/streptavidine) ou un fragment d'anticorps (par exemple un anticorps simple chaîne, un domaine hypervariable d'anticorps isolé) qui se lie spécifiquement à la protéine traduite à partir du gène BCMO1.

Une telle analyse peut être effectuée par une multitude de techniques bien connues de l'Homme du métier, incluant, sans être limitées à, un test immunoenzymatique (EIA), un test radio-immunologique (RIA) ou un test ELISA.

Des anticorps polyclonaux peuvent être préparés par l'immunisation d'un animal adéquat tel que la souris ou le lapin, avec la protéine codée par le gène BCMO1 ou un fragment. Le titre d'anticorps dans l'animal immunisé peut être surveillé dans le temps par des techniques standard telles qu'un ELISA utilisant un polypeptide immobilisé. A un moment approprié après l'immunisation, c'est-à-dire quand le titre de l'anticorps spécifique est au plus haut, les cellules productrices de l'anticorps peuvent être obtenues à partir de l'animal et utilisées pour préparer des anticorps monoclonaux par des techniques standards, telles que la technique de l'hybridome décrite par KOHLER et MILSTEIN (Nature, vol. 256, p :495-497, 1975), la technique de l'hybridome EBV (Cole et al. In monoclonal antibodies and cancer therapy, Alan R. Liss, Inc., p77-96, 1985) ou la techniques des triomes. La technologie pour produire des hybridomes est bien connue (voir par exemple Current protocols in Immunology, Coligan et al. ed., John Wiley & Sons, New York, 1994). Les cellules d'hybridomes produisant l'anticorps monoclonal désiré sont détectées par recherche, dans les surnageants de culture des hybridomes, d'anticorps qui se lient au polypeptide d'intérêt, par exemple en utilisant le test standard d'ELISA.

Le procédé selon la divulgation peut également comprendre une étape supplémentaire b'), après l'étape b), de comparaison du niveau d'expression du gène BCMO1 de l'échantillon biologique avec le niveau d'expression de référence du gène BCMO1 d'un témoin.

Un niveau significativement moins élevé d'expression du gène BCMO1 dans l'échantillon biologique comparé au niveau d'expression de référence du témoin est une indication que l'animal dont est issu l'échantillon biologique possède une viande plus colorée que le témoin.

A l'inverse, un niveau significativement plus élevé d'expression du gène BCMO1 dans l'échantillon biologique comparé au niveau d'expression de référence du témoin est une indication que l'animal dont est issu l'échantillon biologique possède une viande moins colorée que le témoin.

Par sous-expression du gène BCMO1, on entend selon la présente divulgation un niveau d'expression dans un échantillon biologique qui est moins important que l'erreur standard du test employé pour mesurer cette expression, et qui est de préférence au moins 1,25 fois moins important que le niveau d'expression de référence dudit gène, de préférence au moins 1,5 fois moins important que le niveau d'expression de référence dudit gène, plus préférentiellement au moins 1,75 fois moins important que le niveau d'expression de référence dudit gène et encore plus préférentiellement au moins 2 fois moins important que le niveau d'expression de référence dudit gène.

Par niveau d'expression « de référence » du gène BCMO1, on entend selon la présente invention, le niveau d'expression dudit gène dans un échantillon biologique d'un animal d'une même espèce servant de témoin pour la couleur de la viande. Il est rappelé que les valeurs de L*, a* et b* des témoins sont susceptibles d'être différentes selon les espèces animales, mais également en fonction de l'âge ou de l'origine génétique de l'animal ; L'homme du métier saura déterminer facilement pour chaque espèce les valeurs de L*, a* et b* au-dessus ou en-dessous desquelles il souhaite différencier ou sélectionner les animaux, grâce à la littérature ou tout simplement en testant le témoin avec un spectrocolorimètre avec le système CIE L*a*b*. Le tableau 1 présenté ci avant donne des exemples de valeurs de L*a*b* d'animaux témoins.

De préférence, le niveau d'expression «de référence» est déterminé à partir d'un échantillon témoin (un échantillon biologique d'un animal d'une même espèce présentant une couleur de la viande au-dessus ou en-dessous desquelles l'Homme du métier souhaite différencier ou sélectionner les animaux). Le niveau d'expression « moyen » du gène BCMO1 est déterminé à partir de différents échantillons témoins.

Ainsi par exemple pour le poulet, le témoin est une viande avec une valeur du b* voisine de 10, une valeur du a* voisine de 0 et une valeur du L* voisine de 49. Par valeur « voisine », on entend selon la présente divulgation,
- pour b* une valeur de plus ou moins 5 points (soit entre 5 et 15 pour le poulet), avantageusement plus ou moins 2 points (soit entre 8 et 12), avantageusement plus ou moins 1 point (soit entre 9 et 11) ;
- pour a* une valeur de plus ou moins 3 points (soit entre -3 et 3 pour le poulet), avantageusement plus ou moins 2 point (soit entre -2 et 2), avantageusement plus ou moins 1 point (soit entre -1 et 1) ;
- pour L* une valeur de plus ou moins 7 points (soit entre 42 et 56 pour le poulet), avantageusement plus ou moins 5 points (soit entre 44 et 54), avantageusement plus ou moins 2 points (soit entre 47 et 51), avantageusement plus ou moins 1 point (soit entre 48 et 50) ;

Selon un autre aspect, la présente divulgation concerne l'utilisation d'un composé qui inhibe spécifiquement l'expression du gène BCMO1 pour l'obtention d'animaux présentant une viande plus colorée.

De manière préférée selon la présente divulgation, ledit composé est un oligonucléotide, sélectionné dans le groupe comprenant des molécules d'ADN et d'ARN anti-sens, des ribozymes, des SiRNA et des aptamères.

Concernant les ADN anti-sens, les oligodeoxyribonucleotides dérivés du site d'initiation de la traduction sont préférés.

Les molécules d'ADN et d'ARN anti-sens, et les ribozymes selon la présente invention peuvent être obtenus par tout procédé connu de l'Homme du métier pour la synthèse des molécules d'ARN. Ceci inclut les techniques de synthèse chimique d'oligodcoxyribonucleotides bien connues de l'Homme du métier telles que par exemple la synthèse chimique sur phase solide phosphoramidite. De manière alternative, les molécules d'ARN peuvent être générées par transcription in vitro et in vivo de séquences d'ADN codant pour l'ARN anti-sens.

De telles séquences d'ARN peuvent être incorporées dans une grande variété de vecteurs qui incorporent des promoteurs de polymérase ARN appropriés tels que les promoteurs de polymérase T7 ou SP6. De manière alternative, les constructions d'ADNc anti-sens qui synthétisent les ARN anti-sens, de manière constitutive ou inductible, selon le promoteur utilisé, peuvent être introduites de manière stable dans des lignées cellulaires.

De manière encore préférée, ledit oligonucléotide est un SiRNA (ARN interfèrent de petite taille). Ledit SiRNA peut être généré à partir de la séquence génétique du gène BCMO1. De préférence la longueur dudit SiRNA est comprise entre 15 et 25 paires de bases et plus préférentiellement entre 19 et 24 paires de bases. A titre d'exemple on peut citer les SiRNA sélectionnés dans le groupe comprenant SEQ ID N°3: 5'-CUAUCGCCCCAUCACAUAAGA(séquence sens) et SEQ ID N°4: AUGAUAGCGGGGUAGUGUAUU-5' (séquence antisens).

Ledit oligonucléotide peut être administré seul ou en association avec un vecteur. Dans sa définition la plus large, un « vecteur » est tout type de véhicule capable de faciliter le transfert du SiRNA aux cellules.

A titre d'exemple, ledit oligonucléotide peut être associé à un polymère cationique non lipidique (WU and WU, J. Biol. Chem., vol. 263, p : 14621-4, 1988) ou des liposomes (BRIGHMAN et al. Am. J. Med. Sci., vol. 298, p : 278-81, 1989) afin de former des complexes améliorant l'absorption par les cellules.

### LEGENDE DES FIGURES

**Figure 1** **:** Profils de vraisemblance du QTL (estimé par le logiciel QTLMAP) pour a* et b* sur GGA11, dans la phase de primo-localisation (courbes pointillées Nadaf *et al.,* 2007) et après raffinement (courbes pleines).
**Figure 2** **:** Profil de vraisemblance du QTL (estimé par QTLExpress) pour la mesure du jaune (b*), avec ou sans le pH ultime en covariable.
**Figure 3** **:** Position du gène BCMO1 et des marqueurs flanquants sur les cartes physiques et génétiques.
**Figure 4** **:** Profils de vraisemblance du QTL pour les valeurs de rouge (a*) et jaune (b*) (courbes inférieures) et du eQTL pour l'expression brute ou relative par rapport au 18S (courbes supérieures) de BCMO1 sur GGA11
**Figure 5** **:** Statistique de test du QTL (F) pour la mesure de b* et celle de b* corrigée pour la variation d'expression de BCMO1 sur GGA11.
**Figure 6** **:** Fragments contenant les mutations candidates (région promotrice :SEQ ID N°2 ; exon 9 :SEQ ID N°5 ; exon 11 SEQ ID N°6) :
   en italique : SNP observés dans la population
   entre crochets : insertion/délétion observée dans la population
   en gras : mutations candidates (à l'état hétérozygote chez les 5 mâles F1)
   en grisé : couple d'amorces permettant l'amplification du fragment porteur des deux mutations candidates
**Figure 7** **:** Courbes de fusion du fragment amplifié sur plusieurs individus de génotypes différents :
   - individu homozygote A/A (mésappariement avec la sonde) : pic de fluorescence à 56 °C
   - individu homozygote G/G (appariement parfait avec la sonde) : pic de fluorescence à 63 °C
   - individu hétérozygote A/G : pics de fluorescence à 56 °C et 63°C.
**Figure 8** **:** Courbes de fusion du fragment amplifié sur plusieurs individus de génotypes différents :
   - individus homozygotes A/A (mésappariement avec la sonde) : pic de fluorescence à 63 °C
   - individus homozygotes G/G (appariement parfait avec la sonde) : pic de fluorescence à 68 °C
   - individus hétérozygotes A/G : pics de fluorescence à 63 °C et 68°C.
**Figure 9** (a et b): Distribution du niveau absolu d'ARNm de *BCMO1* (a) et de la coloration jaune de la viande (b) dans l'ensemble de la population F2 ou en fonction du génotype des animaux pour les 2 SNP.
**Figure 10** **:** Effet des haplotypes AN57A et GN57G sur l'activité luciférase de pGL2-BCMO1-Luc dans les deux types cellulaires LMH et HuH-7. Les cellules ont été transfectées transitoirement avec 800 ng de plasmide contenant la région -734 à +97 pb du promoteur du gène *BCMO1* poulet contrôlant le gène rapporteur luciférase et 20 ng d'un plasmide de référence contenant le gène de référence β-galactosidase. L'activité de la luciférase est normalisée par l'activité β-galactosidase et est relative à la condition AN57A. **: P < 0.01.

### EXEMPLES

### EXEMPLE 1 : mise en évidence du gène BCMO1

### 1. RAFFINEMENT DE LA REGION QTL A L'AIDE DE NOUVEAUX MARQUEURS.

En 2007 a été identifiée une région QTL localisée sur le chromosome 11 (GGA 11) du poulet, contrôlant la coloration jaune (estimée par la mesure du b*) et dans une moindre mesure rouge (estimée par la mesure du a*) de la viande du filet (Nadaf et al., BMC Genomics 2007, 8 :155). Cette région a été mise en évidence grâce à l'analyse d'un croisement de seconde génération (F2) entre deux lignées divergentes sélectionnées à l'INRA (Institut National de la Recherche Agronomique - Unité de Recherches Avicoles) sur la vitesse de croissance. Comme rapporté par Nadaf et *al.* (2007), ces deux lignées diffèrent pour la croissance et la composition corporelle, mais aussi pour les caractéristiques du filet. Ainsi la lignée à croissance rapide (CR) présente une viande moins colorée (valeurs du b* et du a* plus faibles) que la lignée à croissance lente (CL). Les premières analyses sur ce croisement F2 ont permis d'identifier deux QTLs significatifs pour les mesures b* et a*, aux positions respectives de 62 et 68 cM sur GGA11. Ces deux QTLs étant proches et les mesures de a* et b* corrélées dans la population étudiée (corrélation phénotypique de 0.48 et génétique de 0.74), il est vraisemblable qu'il s'agisse d'un même QTL (et donc gène) contrôlant ces deux caractères. Cette région était largement significative, mais peu précise au niveau de la localisation, avec une taille de 32 CM si bien qu'elle comprenait un nombre de gènes trop important (au total 146) pour permettre l'identification d'un gène candidat particulier.

De nouveaux marqueurs ont donc été typés sur GGA11, permettant de mieux couvrir la partie distale du chromosome (Tableau 2) et préciser la position du QTL.

**Tableau 2 : Ensemble des marqueurs typés sur GGA 11**

| **Marqueur** | **Position (cM)** |
|---|---|
| MCW097 | 18 |
| ADL210 | 54 |
| SEQALL0528 * | 56 |
| ADL308 | 69 |
| SEQALL0529 * | 70 |
| MCW230 * | 88 |

| | |
|---|---|
| * marqueurs développés pour la phase de raffinement | |

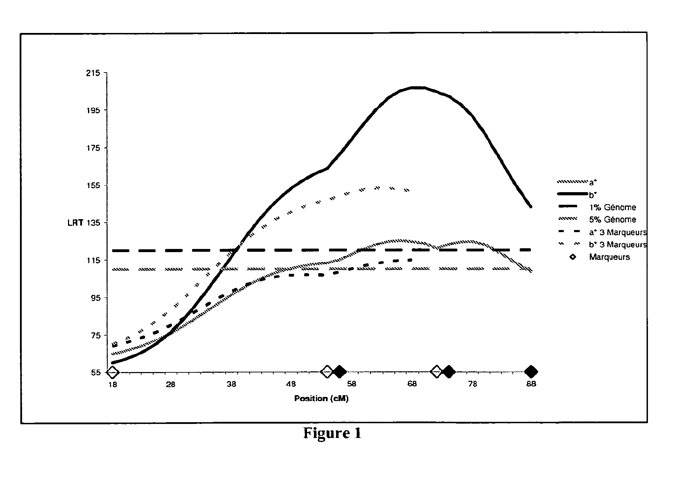

Par rapport aux premières analyses, l'ajout de nouveaux marqueurs a augmenté le degré de signification du QTL pour la mesure du rouge (a*) (LRT de 127 vs 115) et surtout du jaune (b*) (205 vs 153) (**Figure 1**). L'intervalle de confiance a également été diminué, principalement pour la mesure b* en passant de 32 à 14 cM (52-88cM versus 62-76cM selon la méthode de 2 LOD).

Les résultats de profils de vraisemblance du QTL (estimé par le logiciel QTLMAP) pour a* et b* sur GGA11, dans la phase de primo-localisation (courbes pointillées Nadaf et *al.,* 2007) et après raffinement (courbes pleines) sont présentés à la **Figure 1****.**

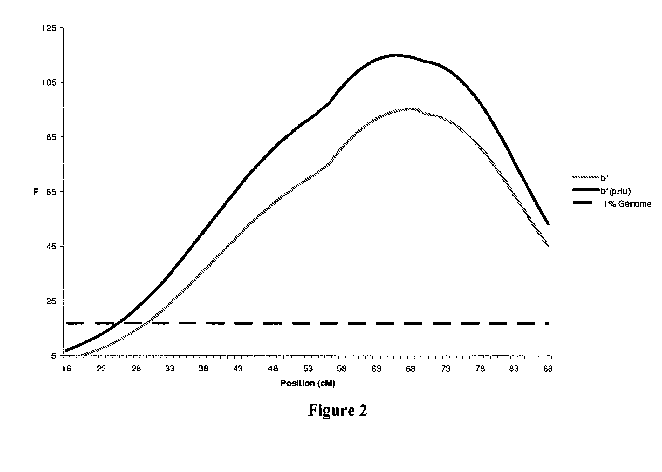

Les mesures de couleur, et en particulier le b*, peuvent être influencées par l'acidité de la viande. Ainsi au niveau du croisement F2, la corrélation entre b* et pH ultime (pHu) est de -0.32 au niveau phénotypique et de -0.48 au niveau génétique. Afin de mieux caractériser l'effet du QTL, le pHu a été introduit en covariable dans le modèle d'analyse. Comme rapporté sur la **Figure 2**, le niveau de signification est alors augmenté. Ce résultat montre que le QTL a un effet indépendant du pH ultime sur la coloration jaune de la viande, ce qui oriente la recherche des gènes candidats sous-jacents.

Le profil de vraisemblance du QTL (estimé par QTLExpress) pour la mesure du jaune (b*), avec ou sans le pH ultime en covariable est présenté à la **Figure 2****.**

Les effets estimés avec QTLMAP (**Tableau 3**) montrent que toutes les familles de père semblent hétérozygotes au QTL pour la coloration jaune. Pour ce caractère, les effets du QTL sont très homogènes entre familles, et expliquent en moyenne 32% de la variance phénotypique. Pour la couleur rouge, même si les différents pères apparaissent hétérozygotes, les effets sont moins prononcés et expliquent en moyenne environ 17% de la variance. Ces analyses confirment également que sur l'ensemble des familles étudiées, les allèles augmentant la coloration jaune ou rouge proviennent de la lignée CL, qui en effet présente une viande plus colorée.

**Tableau 3 : Effets du OTL dans chaque famille de père**

| | **Effet du QTL intra père*** | |
|---|---|---|
| **Père** | **a* (127_66 cM**)** | **b* (205_68 cM**)** |
| 634 | 0,219 | 0,336 |
| 657 | 0,125 | 0,247 |
| 687 | 0,105 | 0,343 |
| 780 | 0,233 | 0,316 |
| 805 | 0,15 | 0,342 |

| | | |
|---|---|---|
| * Un signe positif signifie que l'allèle augmentant la couleur vient de la lignée CL ** LRT statistique _ Position | | |

Afin de préciser l'effet du QTL chez les animaux F2, 3 groupes ont été constitués selon leur génotype estimé au QTL (soit QQ, qQ et qq). Ce dernier a été estimé sur la base des probabilités de transmission des haplotypes calculées par QTLExpress, qui permettent de reconstituer l'origine grand-parentale des allèles reçus par le descendant F2 à la position du QTL. Le génotype (QQ, Qq ou qq) a été estimé en faisant l'hypothèse d'une fixation complète des allèles au QTL dans les souches grand-parentales, celle à croissance rapide (CR) transmettant l'allèle q (effet négatif sur la couleur) et celle à croissance lente (CL) l'allèle Q (effet positif sur la couleur). Au total, le génotype au QTL a pu être reconstitué avec une bonne précision (p>0.99) sur un total de 602 animaux. Un effet significatif du génotype au QTL sur les mesures de b* et a* a été mis en évidence : la différence entre les groupes QQ et qq est d'environ 1 et 0.5 écart-type phénotypique, respectivement pour les valeurs de b* et de a*. Cette différence est légèrement plus marquée pour la mesure du b* corrigée pour le pH ultime (**Tableau 4**). Ces analyses montrent que le génotype hétérozygote est intermédiaire entre les deux homozygotes (qq et QQ), ce qui indique un effet additif des allèles au QTL.

**Tableau 4 : Valeurs moyennes des mesures de couleur (centrées sur les effets lot et sexe) en fonction du génotype au QTL**

| **Génotype*** | **Caractère** | **N** | **Moyenne** | **écart-type** |
|---|---|---|---|---|
| qq | b* | 144 | -0.75 | 1.35 |
| | a* | 146 | -0.35 | 0.83 |
| Qq | b* | 275 | 0.03 | 1.45 |
| | a* | 276 | 0.08 | 0.98 |
| QQ | b* | 137 | 0.78 | 1.32 |
| | a* | 139 | 0.22 | 0.82 |

| | | | | |
|---|---|---|---|---|
| *q est l'allèle transmis par la lignée CR et Q celui transmis par la lignée CL. | | | | |

### 2. ETUDE DE LA VARIATION D'EXPRESSION D'UN GENE CANDIDAT : BCMO1

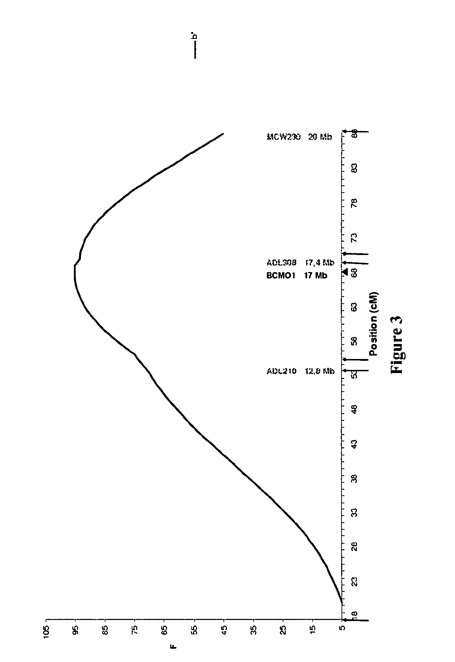

L'ajout de nouveaux marqueurs a permis de réduire l'intervalle de confiance du QTL, qui comprend après raffinement environ 27 gènes. Un des gènes candidats identifié au proche voisinage du pic de vraisemblance pour le QTL (obtenu à environ 68 cM, **Figure 3**) est le gène BCMO1 (Numéro d'accession GO : 0003834 ; GenBank : AJ271386). Le gène BCMO1 (Béta,béta-carotène 15,15'-monooxygénase), anciennement BCDO1 (Béta-carotène dioxygénase 1), est présent sur la séquence du génome de la poule sur l'assemblage du chromosome 11 entre les bases 17 085 000 et 17 105 000. Le transcrit a une taille de 3 046 paires de bases (pb), pour une protéine de 526 acides aminés.

### 2.1 Caractérisation du niveau d'expression de BCMO1 dans le muscle Pectoralis major (filet) des lignées parentales CL et CR

### 2.1.1 Dispositifs et prélèvement des échantillons

Les animaux étudiés étaient issus de deux dispositifs distincts :
- Les populations pures correspondant aux deux lignées divergentes pour la croissance (CL et CR) abattues à 1, 3, 5, 7, 9 et 11 semaines (n = 6 animaux par âge et par lignée),
- L'ensemble des descendants d'un père F1 du dispositif de détection de QTL (soit 134 animaux, tous abattus à 9 semaines).

Pour chaque individu, un échantillon de muscle *Pectoralis major* du filet a été prélevé quelques minutes après la mort de l'animal, congelé immédiatement dans l'azote liquide, puis conservé à -80°C.

### 2.1.2 Préparation des ARN

L'extraction des ARN totaux a été réalisée à l'aide de la solution RNA Now (Ozyme, France) selon le principe de Chomzynski and Sacchi (1987). Cent milligrammes de tissu broyé ont été homogénéisés en présence de RNA Now (1 ml) à l'aide d'un broyeur à bille (Rescht MM 301). Après addition de 200 µl de chloroforme pur, les homogénats ont été agités manuellement pendant 1 minute puis placés 5 minutes dans la glace. Après centrifugation (10 minutes, 12 000g), la phase supérieure contenant les ARN a été prélevée et mélangée à un volume égal d'isopropanol. Après 5 minutes de précipitation dans la glace, les ARN ont été centrifugés (10 min, 12 000g) et les culots d'ARN obtenus ont été lavés successivement deux fois à l'éthanol 70% (1 ml) puis centrifugés à 5000 g (5 minutes). Enfin, ils ont été séchés à l'air libre pendant 10 minutes et les ARN ont été remis en suspension dans 30 µl d'eau stérile. Les échantillons d'ARN ont été dosés par spectrophotométrie à 260 nm et leur contamination éventuelle par des protéines estimée à 280 nm. L'intégrité des ARN a par ailleurs été vérifiée par électrophorèse sur gel d'agarose 1% (préparé dans du tampon TBE 0,5X en présence de bromure d'éthidium). Les solutions d'ARN (1 µg/µl) ont été stockées à -80°C.

Afin d'éliminer toute trace d'ADN génomique, les solutions d'ARN ont été traitées à la DNAse (kit DNA-free, Ambion). Dix microlitres d'ARN (1 µg/µl) ont été incubés à 37°C pendant 20 minutes en présence de 1 µl de Tampon DNase et 1 µl de l'enzyme DNase. Puis 1 µl d'inhibiteur de l'activité DNase a été ajouté afin d'arrêter la réaction. Après 2 minutes à température ambiante, les échantillons ont été centrifugés (1 min, 10 000g) afin de précipiter l'inhibiteur de l'enzyme. Le surnageant a été prélevé et stocké à -80°C.

### 2.1.3 Transcription inverse

Les ADN complémentaires (ADNc) ont été obtenus par transcription inverse à partir de 5 µl d'ARN traités à la DNase mélangés à 5 µl d'H₂O ultra pure, 1 µl d'hexamères nucléotidiques (Random primers 0,5 µg/µl, Promega) et 1 µl de désoxynucléotides (dNTP mix 10 mM, Sigma). Une solution contenant 2 µl de DTT (0,1 M), 1 µl de RNase out (inhibiteur de RNase), 1 µl d'enzyme SuperScript II (SuperScript ^{™} Reverse transcriptase II, Invitrogen 40 units/µl) et 4 µl de tampon (5X) de la reverse transcriptase (nécessaire pour établir les conditions optimales à la réaction) a été ajoutée à chaque échantillon et la réaction de transcription inverse effectuée pendant 50 minutes à 42°C. Dix microlitres d'H₂O ultrapure ont ensuite été ajoutés. Les ADNc ainsi obtenus ont été stockés à -20°C.

### 2.1.4 Détermination des niveaux d'expression d'ARNm par RT-PCR quantitative

Les réactifs utilisés sont respectivement le qPCR^{™} Mastermix Plus pour la réaction SYBR Green I et le qPCR^{™} Mastermix Plus pour la réaction TaqMan (Eurogentec S.A., Angers, France). Le SYBR green est utilisé pour visualiser l'amplification de l'ADNc du gène BCMO1 et la sonde TaqMan pour l'ARN ribosomal 18S. Ces mélanges sont constitués de dNTPs, d'une ADN polymérase Hot Start activée à 95°C, d'une Uracile-N Glycosylase, de 5 mM de MgCl₂, d'un stabilisateur, d'une référence passive, le ROX, et du SYBR green ou de la sonde TaqMan. Les produits (ADNc) issus de la transcription inverse ont été dilués au 1/50^{ème} pour BCMO1 et au 1/5000^{ème} pour l'ARNr18S. Les échantillons ont été déposés en triple sur des plaques de 96 puits. Chaque plaque contenait un contrôle négatif (eau) déposé en double ainsi qu'une référence (mélange réalisé à partir de 30µg d'ARN de 20 échantillons) permettant la comparaison des différentes plaques de PCR. La réaction de PCR en temps réel a été réalisée à partir de 5 µl d'ADNc en présence de 5,5 µl d'H₂O ultra pure, 12,5 µl de Mix, 1 µl d'amorce sens (5'-AACAAAGAAGAGCATCCAGAGCC-3', SEQ ID N°34), 1 µl d'amorce anti-sens (5'-GCCA.AGCCATCAAACCAGTG-3', SEQ ID N°35) pour la réaction SYBR green d'amplification de BCMO1 et de 6,5 µl d'eau ultra pure, 12,5 µl de Mix et de 1 µl d'amorces sens et antisens et de sonde (Human 18S rRNA, Applied Biosystems, Warrington, UK) pour la réaction TaqMan d'amplification de l'ARNr 18S. La réaction de PCR est réalisée dans un thermocycleur (ABI Prism 7000, Applied Biosystems), dans les conditions suivantes : une dénaturation initiale (95°C pendant 10 minutes), puis 40 cycles d'amplification comprenant une étape de dénaturation (95°C, 15 secondes) suivi par une phase d'hybridation et d'élongation (1 minute à 62°C ou 60°C pour BCMO1 et ARNr 18S, respectivement). Afin de prouver la spécificité et l'unicité du fragment d'ADNc de BCMO1 amplifié, une étape supplémentaire de dissociation a été réalisée à la fin des 40 cycles (95°C, 15 secondes ; 62°C ou 60°C, 20 secondes ; 95°C, 15 secondes). L'efficacité (E) de la PCR pour le gène d'intérêt a été déterminée en réalisant des courbes de dilution standard. Elle était proche de 100 %.

Une analyse automatique est alors réalisée par le logiciel 7000 System Software et chaque réaction est caractérisé par un Ct correspondant au nombre de cycles nécessaires pour que la fluorescence émise franchisse un seuil de détection prédéfini. Le Ct est inversement proportionnel au nombre de copies du fragment amplifié. Pour chaque échantillon un Ct moyen a été calculé à partir des 3 réplicats mesurés.

Les niveaux d'expression de BCMO1 ont ensuite été calculés selon l'équation de Pfaffl (2001) :

### Quantité d'ARNm BCMO1 dans l'échantillon = ΔCT_{BCMO1} (Référence-Echantillon)

ou exprimés en écart à la quantité d'ARNr18S dans chaque échantillon :

### Quantité d'ARNm BCMO1/18S = ΔCT_{BCMO1} (Référence-Echantillon)-ΔCT₁₈ₛ(Référence-Echantillon)

### 2.1.5 Résultats

Comme attendu, l'expression de l'ARNr 18S n'est affecté ni par la lignée ni par l'âge d'abattage des poulets (**Tableau 5**). Par contre, quel que soit l'âge d'abattage, l'expression de BCMO1 (en niveau absolu ou relatif par rapport à l'ARNr 18S) est supérieure chez les animaux issus de la lignée CR par rapport à CL (P ≤ 0.001). Ceci est cohérent avec la diminution de coloration jaune (et rouge) de la viande observée dans la lignée CR par rapport à la lignée CL (Nadaf et al., 2007).

**Tableau 5 : Niveaux d'expression en ARN dans le muscle P. major pour 18S et BCMO1 (niveaux absolus et relatifs) nour chaque lignée à chanue age (n = 6).**

| | **ARNr 18S** | | **ARNm BCMO1** | | **BCMO1/18S** | |
|---|---|---|---|---|---|---|
| **Lignée** | CR | CL | CR | CL | CR | CL |
| 1 semaine | 0.21 ± 0.28 | 0.30 ± 0.15 | -0.71 ± 0.53 | -2.06 ± 1.38 | -0.92 ± 0.76 | -2.35 ± 1.32 |
| 3 semaines | 0.12 ± 0.51 | 0.21 ± 0.18 | -0.96 ± 1.39 | -2.25 ± 0.79 | -1.09 ± 1.38 | -2.46 ± 0.75 |
| 5 semaines | 0.24 ± 0.33 | 0.09 ± 0.28 | 0.33 ± 0.52 | -0.89 ± 0.29 | 0.08 ± 0.48 | -0.98 ± 0.31 |
| 7 semaines | 0.04 ± 0.14 | 0.14 ± 0.26 | -0.18 ± 1.15 | -1.67 ± 0.52 | -0.23 ± 1.16 | -1.81 ± 0.61 |
| 9 semaines | 0.02 ± 0.10 | 0.28 ± 0.14 | 0.26 ± 0.30 | -1.44 ± 0.80 | 0.24 ± 0.36 | -1.73 ± 0.67 |
| 11 semaines | -0.06 ± 0.41 | 0.12 ± 0.34 | -0.34 ± 0.55 | -1.30 ± 0.76 | -0.28 ± 0.54 | -1.42 ± 0.86 |
| Effet lignée | NS | | *** | | *** | |
| Lignée x âge | NS | | NS | | NS | |

### 2.2 Détection d'un e-QTL pour la variation d'expression génique de BCMO1

La quantité d'ARNm a été mesurée dans le muscle Pectoralis *major* chez les 134 descendants du père F1 780, dont le statut hétérozygote au QTL de couleur a été confirmé par les nouvelles analyses. Les corrélations phénotypiques entre niveaux d'expression de BCMO1 (estimés en valeur brute ou en valeur relative par rapport à l'expression du 18S) ont confirmé un lien entre niveau d'expression du gène et indicateurs de couleur, avec des corrélations négatives de -0.47 et -0.46 (p<0.0001) entre quantité d'ARN et valeurs de a* et de b*, respectivement. Ces corrélations étaient toujours négatives mais moins fortes avec l'expression relative de BCMO1 par rapport au 18S (corrélations de -0.25 et -0.28 respectivement, p<0.003). **Ces résultats indiquent, qu'au sein des descendants de cette famille, une augmentation de l'activité du gène BCMO1 est associée à une diminution de la coloration rouge et jaune du muscle du filet.**

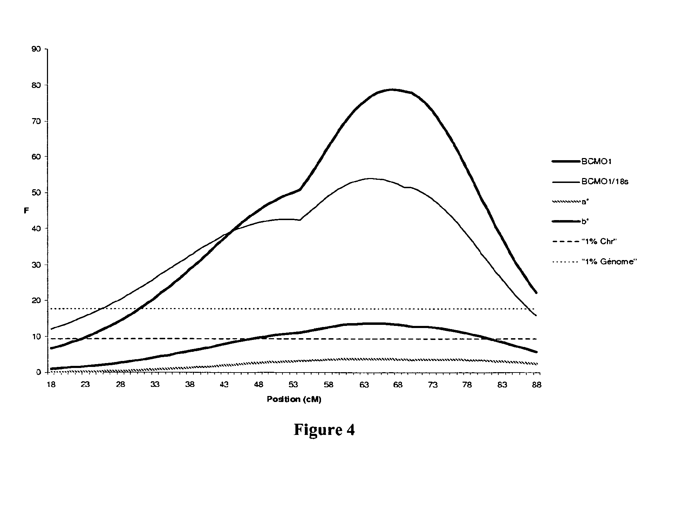

La recherche de QTL contrôlant la mesure d'expression de BCMO1 a été faite par une analyse F2 avec le logiciel QTLExpress. Un QTL contrôlant le niveau d'expression « brute » du gène a été identifié sur le chromosome 11 en position 67 cM (**Figure 4**), c'est-à-dire à la « même » position que celui détecté pour la mesure du jaune. La valeur de la statistique F est extrêmement significative (égale à 79, pour une valeur limite à 1% au niveau du chromosome de 9.5). Pour l'expression relative par rapport au 18S, les résultats sont très proches mais le niveau de signification du QTL est moins marqué. L'allèle transmis par la lignée CR augmente le niveau d'expression du gène, avec un effet additif estimé à 0.93 ± 0.1 pour l'expression brute et 1.05 ± 0.14 pour l'expression relative. Ces effets correspondent à une différence du niveau d'expression de BCMO1 entre les 2 groupes d'homozygotes QQ et qq de l'ordre de 2 écart-types phénotypiques.

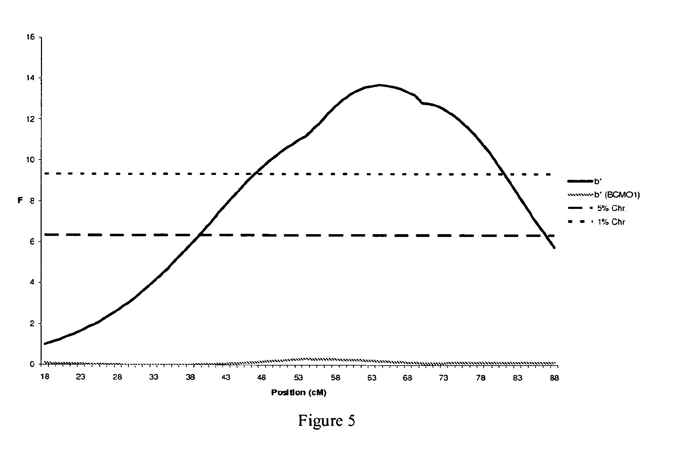

De nouvelles analyses de détection de QTLs ont aussi été faites sur les variations des mesures de a* et b* indépendantes de la variation d'expression de BCMO1 (c'est-à-dire en introduisant l'expression de BCMO1 en covariable dans le modèle). Dans ce cas, aucun QTL significatif pour la couleur jaune (ou rouge) n'est retrouvé sur le chromosome 11 **(****Figure 5****). Ce résultat montre que l'effet du QTL contrôlant la couleur passe par une variation de l'expression de BCMO1.**

### 3. RECHERCHE DE POLYMORPHISME AU SEIN DE BCMO1

D'après les analyses QTL, les 5 pères du dispositif F2 sont hétérozygotes au QTL. La mutation causale doit donc être présente à l'état hétérozygote chez tous ces individus. De plus, nos résultats montrent que l'effet du QTL passe par une variation du niveau d'expression de BCMO1, ce qui suggère que la (ou les) mutation causale est située dans la région promotrice du gène. L'ensemble de la région codante du gène, ainsi que la zone en 5' du gène dans laquelle se situe la région promotrice, a donc été séquencé sur les 5 mâles pour rechercher une mutation répondant au critère d'hétérozygotie. Nous avons amplifié par PCR l'ensemble de la région codante du gène grâce à des amorces spécifiques choisies sur la séquence de la poule, puis nous avons séquencé les fragments obtenus.

### 3.1 Définition des couples d'amorces

Des couples d'amorces ont été définis à partir de la séquence génomique de la poule (2) avec le logiciel LightCycler Probe Design2 (Roche), pour amplifier chacun des exons. Neuf couples ont été choisis dans les introns, un couple a été dessiné pour amplifier les exons 5 et 6 et l'intron 5, qui est court, et un couple a été défini dans la région promotrice (**Tableau 6**).

**Tableau 6 : Amorces et conditions d'amplifications utilisées pour le séquençage du gène BCMO1.**

| **Région ciblée** | **Amorce U** | **Amorce L** | **Taille (pb)** | **Tm pCR** |
|---|---|---|---|---|
| exon 10 | SEQ ID N°7 ACAATGTAATGAAGTGGTATCTATG | SEQ ID N°8 TGCAAGCTCCTTTTGCTC | 538 | 55°C |
| exon 11 | SEQ ID N°9 AGATTCCTAGCAAACAAGACT | SEQ ID N°10 TTTGTGTCGTTATATGGTTGTTC | 366 | 52°C |
| exon 1 | SEQ ID N°11 AAGATCAACTGTTATAAGTTGTCG | SEQ ID N°12 CTGCAAGTATTAGAAGAGATTGTCA | 261 | 55°C |
| exon 2 | SEQ ID N°13TATGAAGCCTATGCCCTTAGT | SEQ ID N°14TGAATTGGGAACATAAGACACC | 286 | 55°C |
| exon 3 | SEQ ID N°15 GAAACGTAGTGTGAAATGTGAT | SEQ ID N°16 ATATTTGTATTCACGTGCCAAGA | 270 | 55°C |
| exon 4 | SEQ ID N°17 CTGCAGGTACAGCTTCT | SEQ ID N°18 GCCAGGAAATGGGAGGAAATA | 302 | 55°C |
| exons 5 et 6 | SEQ ID N°19 ATGGGCATTTGCAGAGT | SEQ ID N°20 GAGCAGCAACAGTAACAAC | 842 | 55°C |
| exon 7 | SEQ ID N°21 ATTTCCTATGTAACTGTTTGCTG | SEQ ID N°22 CAACTGCTCATCCACCC | 403 | 55°C |
| exon 8 | SEQ ID N°23 ATTAGAAGCACATTACATTTCCAG | SEQ ID N°24 ACATCTAAGTGTAGGTGTACAAG | 204 | 52°C |
| exon 9 | SEQ ID N°25 TACCACTTTTGAACAAACTGCC | SEQ ID N°26 TCTGGGTTGGTGACTGCAGA | 423 | 55°C |
| région promotrice | SEQ ID N°27 CTCTGCAATCACATGCTTTAT | SEQ ID N°28 TCTGGATGCTCTTCTTTGTTT | 858 | 52°C |

### 3.2 Amplification PCR

Les 11 fragments couvrant la totalité de la région codante du gène BCMO1 et de sa région promotrice ont été amplifiés à partir de l'ADN des 5 mâles du dispositif. Les amplifications PCR ont été réalisées pour chaque fragment dans un mélange réactionnel (15 µl final) contenant 25 ng d'ADN génomique, 0.4 µM d'amorces, 0.25 unités de Taq polymerase (Go Taq, Promega), 2 mM de MgCl2 et 0.2 mM de dNTP (Promega) sur des thermocycleurs 9700 (Applied Biosystems). La première dénaturation de 5 minutes était suivie de 38 cycles composés de 30 s de dénaturation à 94°C, 30 s d'hybridation à 55°C ou 52°C, et 30 s d'élongation à 72°C, puis de 10 minutes d'élongation finale à 72°C.

### 3.3 Séquençage

Les réactions de séquence ont été réalisées pour chaque sens en utilisant chacune des amorces ayant servi à l'amplification et le kit diChloro Rhodamine Prism AmpliTaq FS Big Dye Terminator V3.1 (Applied Biosystems). Les séquences ont été analysées sur un séquenceur ABI 3100 (Applied Biosystems). L'analyse des chromatogrammes a ensuite été réalisée avec le logiciel Chromas (Technelysium Pty Ltd).

### 3.4 Résultats

Les séquences obtenues sur les 5 mâles F1 ont permis d'observer 23 SNP, dont 4 SNP dans la région codante, et 4 insertions/délétions. Six de ces 23 mutations sont observées à l'état hétérozygote chez les 5 pères, caractéristique indispensable pour obtenir le statut de candidate. Elles sont décrites sur la figure 6 : une de ces mutations est située dans l'exon 11, 2 dans l'intron 8 et 1 dans l'intron 9, et 2 dans la région promotrice. Ces dernières sont les candidates les plus fortes, sachant que la mutation causale est associée à des différences de niveau d'expression du gène. Ces 2 mutations candidates dans le promoteur SEQ ID N°2 (- CCT*A/G*CCA et - CGT*A/G*GGA) ont également été séquencées chez 1 animal F0 de la lignée CL et 2 animaux F0 de la lignée CR qui sont, comme attendu, homozygotes à ces 2 mutations et de type AN₅₇A pour la lignée CR et GN₅₇G pour la lignée CL. Par ailleurs, quelques séquençages ont aussi été réalisés sur des animaux F2 dont le statut au QTL avait été préalablement estimé par le logiciel QTLMAP. Il y a sur les 6 animaux F2 testés parfaite adéquation entre le statut supposé au QTL et le génotype défini par ces 2 mutations : les animaux ayant reçu 2 allèles grand-parentaux d'origine CR sont bien de génotype homozygote AN₅₇A alors que ceux ayant reçu 2 allèles grand-parentaux d'origine CL sont homozygotes de type GN₅₇G.

### Conclusion

Via leur effet sur l'expression de BCMO1 les 2 mutations identifiées dans le promoteur modulent la couleur de la viande dans les teintes jaune et rouge

EXEMPLE 2 : Procédé de sélection de poulets présentant une viande plus colorée selon la présente invention.

Afin de sélectionner des poulets présentant une viande plus colorée que le standard, les étapes suivantes du procédé selon l'invention sont mises en oeuvres :

### 1. Génotypage des mutations candidates.

### 1.1 - Génotypage de la mutation A/G à la position 139 de SEQ ID N°2

Parmi les méthodes utilisables pour déterminer le génotype des animaux à cette mutation (polymorphisme de longueur des fragments de restriction, conformation en simple brin, hybridation allèle-spécifique...), on peut mettre en oeuvre la technique de HRM (High-Resolution Melting) selon le protocole suivant :

Un couple d'amorces est choisi de manière à amplifier le fragment contenant la mutation à tester :
Upper : 5'- GAACTCAGTTAAGTACCTTATCT -3', (SEQ ID N°29)
Lower : 5'- AGCAGCACACCTTTGAATTAAACA -3'. (SEQ ID N°30)

Un troisième oligonucléotide est utilisé en tant que sonde allèle-spécifique :
5'- GCGAAAGTATGGCAGGAATAATTAA-P -3' (SEQ ID N°31).

Les amplifications PCR sont réalisées dans un mélange réactionnel (15 µl final) contenant 25 ng d'ADN génomique, 0.5 µM de l'amorce upper, 0.1 µM de l'amorce lower, 0.25 unités de Taq polymerase et 1X de tampon associé (Go Taq, Promega par exemple), 2 mM de MgCl2 et 0.2 mM de dNTP (Promega par exemple) sur un thermocycleur (9700 Applied-Biosystems, par exemple). La première dénaturation de 5 minutes est suivie de 45 cycles composés de 20 s de dénaturation à 95°C, 20 s d'hybridation à 58°C, et 20 s d'élongation à 72°C, puis de 10 min d'élongation finale à 72°C. Sont ajoutés à 8 µL du mélange 0.4 µM de la sonde allèle-spécifique et 1X d'un agent de marquage (LC Green, Idaho Technology, par exemple). La courbe de fusion est obtenue par une augmentation de la température à 95°C pendant 10 min, puis par diminution de la température à 40 °C, par paliers (10s à 85, 80, 75, 70, 65, 60, 50°C, 2,2°C/s), et chauffage (0.02°C/s) jusqu'à 95 °C sur un thermocycleur LC-480 (Roche) par exemple.

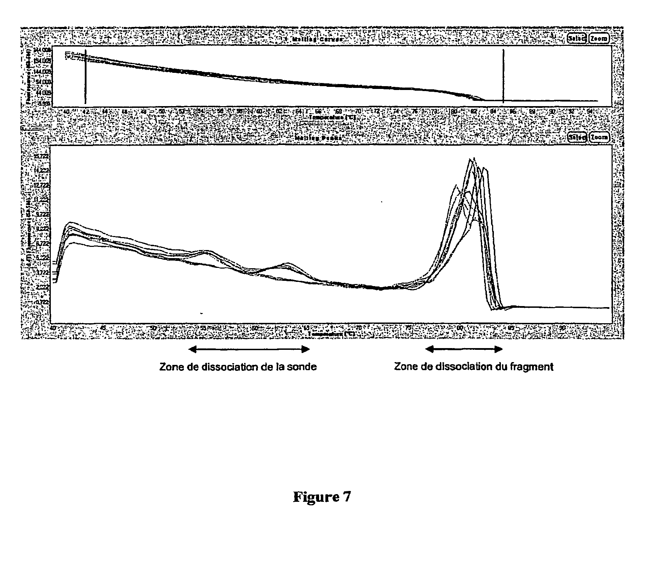

Les résultats sont lisibles de la manière indiquée sur la **Figure 7****.**
- individu homozygote A/A (mésappariement avec la sonde) : pic de fluorescence à 56 °C
- individu homozygote G/G (appariement parfait avec la sonde) : pic de fluorescence à 63 °C
- individu hétérozygote A/G : pics de fluorescence à 56 °C et 63°C.

### 1.2 - Génotypage de la mutation A/G à la position 197 de SEQ ID N°2

Parmi les méthodes utilisables pour déterminer le génotype des animaux à cette mutation (polymorphisme de longueur des fragments de restriction, conformation en simple brin, hybridation allèle-spécifique...), la technique de HRM (High-Resolution Melting) est choisie et mise en oeuvre selon le protocole suivant :

Un couple d'amorces est choisi de manière à amplifier le fragment contenant la mutation à tester :
Upper 5' -AGGCTTCGAGTTGCTGATAACC -3', (SEQ ID N°32)
Lower 5'- AGCAGCACACCTTTGAATTAAACA -3' (SEQ ID N°30).

Un troisième oligonucléotide est utilisé en tant que sonde allèle-spécifique :
5'-CTATTCTACATTCTCCCACGTGTGATCTGATT-P-3' (SEQ ID N°33).

Les amplifications PCR sont réalisées dans un mélange réactionnel (15 µl final) contenant 25 ng d'ADN génomique. 0.5 µM de l'amorce upper, 0.1 µM de l'amorce lower et 0.4 µM de la sonde, 0.25 unités de Taq polymerase et 1X de tampon associé (Go Taq, Promega par exemple), 2 mM de MgCl2, 1X d'agent de marquage (LC Green, Idaho Technology, par exemple) et 0.2 mM de dNTP (Promega par exemple) sur un thermocycleur (LC-480, Roche par exemple). La première dénaturation de 10 minutes est suivie de 50 cycles composés de 1 s de dénaturation à 95°C, 1 s d'hybridation à 58°C, et 10 s d'élongation à 72°C. La courbe de fusion est obtenue par une augmentation de la température à 95°C pendant 1s, puis par diminution de la température à 40 °C et chauffage (0.02°C/s) jusqu'à 95 °C sur le même appareil.

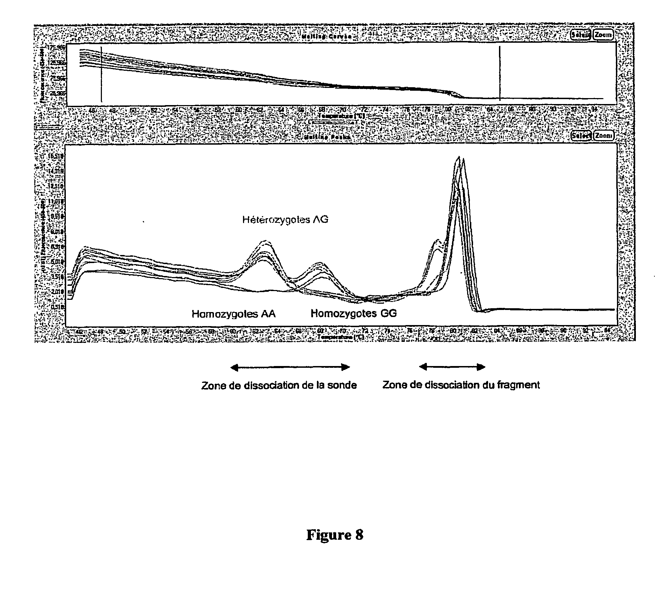

Les résultats sont lisibles de la manière indiquée sur la **Figure 8****.**
- individu homozygote A/A (mésappariement avec la sonde) : pic de fluorescence à 63 °C
- individu homozygote G/G (appariement parfait avec la sonde) : pic de fluorescence à 68 °C
- individu hétérozygote A/G : pics de fluorescence à 63 °C et 68°C.

### 2. Analyse de l'expression du gène BCMO1 dans un échantillon biologique de poulets

Parmi les méthodes utilisables pour déterminer l'expression du gène BCMO1 dans un échantillon biologique, la technique de RTPCR quantitative est choisie et mise en oeuvre selon le protocole suivant :

La réaction de PCR en temps réel est réalisée à partir de 5 µl d'ADNc (issus de la transcription inverse d'ARNm) en présence de 5,5 µl d'H₂O ultra pure, 12,5 µl de Mix (qPCR™ Mastermix Plus), 1 µl d'amorce sens SEQ ID N°34 (5'-AACAAAGAAGAGCATCCAGAGCC-3'), 1 µl d'amorce anti-sens SEQ ID N°35 (5'-GCCAAGCCATCAAACCAGTG-3') pour la réaction SYBR green d'amplification de BCMO1 et de 6,5 µl d'eau ultra pure, 12,5 µl de Mix (qPCR^{™} Mastermix Plus) et de 1 µl d'amorces sens et antisens et de sonde (Human 18S rRNA, Applied Biosystems, Warrington, UK) pour la réaction TaqMan d'amplification de l'ARNr 18S.

La réaction de PCR est réalisée dans un thermocycleur dans les conditions suivantes : une dénaturation initiale (95°C pendant 10 minutes), puis 40 cycles d'amplification comprenant une étape de dénaturation (95°C, 15 secondes) suivie par une phase d'hybridation et d'élongation (1 minute à 62°C ou 60°C pour BCMO1 et ARNr 18S, respectivement).

### 3. Analyse de l'expression de la protéine BCMO1 dans un échantillon biologique de poulets

Parmi les méthodes utilisables pour déterminer l'expression de la protéine BCMO1 dans un échantillon biologique, un test de dosage d'immunologique, incluant un test ELISA utilisant un anticorps spécifique de BCMO1 chez le poulet est choisi et mis en oeuvre.

La production d'un tel anticorps se fait dans le cadre d'un protocole d'immunisation utilisant deux peptides de synthèse distincts spécifiques du gène, les séquences en acides aminés suivantes : METIFNRNKEEHPEP (SEQ ID N°36) et DKDFEVNNKLTSIPTC (SEQ ID N°37).

**EXEMPLE 3 :** Effet des deux mutations candidates (positions 139 et 197 de SEQ ID N°2) sur le niveau d'expression du gène et la coloration jaune de la viande du filet dans le croisement F2 entre lignées CL et CR.

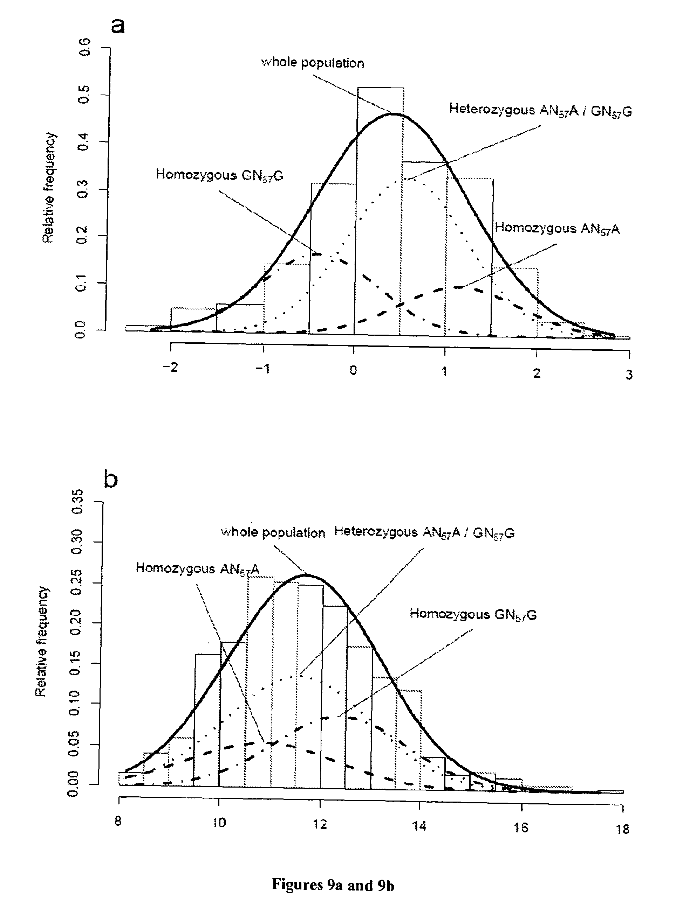

Les deux mutations candidates ont été typées sur l'ensemble des animaux F0, F1 et F2 du dispositif de détection des QTL. Les résultats montrent, qu'au moins dans cette population expérimentale, les deux mutations sont totalement liées: seuls les haplotypes AN₅₇A et GN₅₇G sont observés alors qu'on observe aucun recombinant AN₅₇G ou GN₅₇A chez les animaux F0, F1 ou F2 du dispositif. Le génotypage des animaux F0 révèle des différences de fréquence allélique entre les lignées CL et CR: l'haplotype GN₅₇G est fixé chez les animaux F0 de la lignée CL (n=20), alors que l'haplotype AN₅₇A est plus fréquent mais pas totalement fixé chez les animaux F0 de la lignée CR (parmi les 13 animaux génotypés, 9 sont de type AN₅₇A et 4 de type GN₅₇G). Le génotypage des deux mutations candidates sur la population F2 montre un effet significatif sur le niveau d'expression musculaire de *BCMO1* **(****Fig. 9a****) :** le niveau d'ARN messagers est estimé à 1.17 (n=63) et -0.48 (n=101) chez les homozygotes de type AN₅₇A et GN₅₇G, soit une différence d'environ 2 écart-types entre ces groupes extrêmes (P<0.0001). Les animaux hétérozygotes, de type AN₅₇A/GN₅₇G, présentent un niveau d'expression intermédiaire (0.56, n=203) suggérant un effet additive des mutations. De façon cohérente avec la fonction connue du gène, les résultats confirment un effet significatif des 2 SNP sur le niveau de coloration jaune de la viande du filet (**Fig. 9b**). Une différence de 1.5 unité de couleur jaune (environ 1 écart-type) est observée entre les homozygotes, alors que les hétérozygotes présentent une valeur intermédiaire (b*=10.9, 11.6 et 12.4 chez les homozygotes AN₅₇A (n=128), les hétérozygotes AN₅₇A/GN₅₇G (n=318) et les homozygotes GN₅₇G (n=188), respectivement). Un effet significatif du génotype est aussi mis en évidence sur la coloration rouge du filet avec des valeurs moyennes de a* estimées à 1.3 pour les homozygotes GN₅₇G (n=190), 1.1 pour les hétérozygotes AN₅₇A/GN₅₇G (n=320) et 0.7 pour les homozygotes AN₅₇A (n=129), soit une différence de 0.7 écart-type entre les groupes extrêmes (P<0.0001)

### EXEMPLE 4 : Validation fonctionnelle des 2 SNP liés sur l'activité du promoteur.

Après avoir confirmé l'association entre les deux mutations et le niveau d'expression du gène et la couleur jaune de la viande du filet, l'objectif de cette nouvelle étude était de prouver leur effet fonctionnel sur le niveau d'activité du promoteur. Les effets fonctionnels des deux haplotypes (AN₅₇A et GN₅₇G) sur l'activité du promoteur ont été étudiés par une stratégie de type gène rapporteur.

### Réalisation des constructions plasmidiques :

Le promoteur du gène *BCMO1* du poulet a été amplifié par PCR des bases -734 à +97 en utilisant les amorces suivantes : BCMO1F: 5'-AGCCTGTGATTTCCTCTGC-3' (SEQ ID N°38) et BCMO1R: 5'-TCGCTCTCCTGTGTCCTCTA-3' (SEQ ID N°39). Dans un premier temps, l'amplicon a été sous cloné dans un plasmide PCR3.1 selon la procédure de « TA cloning » (Invitrogen, Carlsbad, CA). Dans un second temps, le fragment cloné dans le plasmide PCR3.1 a été transféré dans un plasmide pGL2 basic (Promega, Madison, WI), en amont du gène rapporteur de la luciférase, pour créer le plasmide pGL2-BCM01-Luc. Des lignées d'hépatome de poulet (LMH) et humaines (HuH-7) ont été transfectées par un vecteur portant le gène rapporteur luciférase gouverné par la région promotrice -734 à +97 du gène *BCM01* composée soit de l'haplotype GN₅₇G ou AN₅₇A.

### Culttrre cellulaire et dosage de l'activité transcriptionnelle

Les cellules hépatiques de poulet (LMH) ont été achetées à ATCC (cat# CRL-2117). Ces cellules ont été cultivées dans un milieu Waymouth's (Invitrogen, Carlsbad, CA) complémenté par 10% de sérum de veau foetal (SVF, Invitrogen, Carlsbad, CA), 100 IU/ml de pénicilline (Invitrogen, Carlsbad, CA) et 100 mg/ml de streptomycine (Invitrogen, Carlsbad, CA). Les cellules hépatiques humaines (HuH-7) ont été cultivées dans du milieu William's (Invitrogen, Carlsbad, CA), avec 10% de SVF, 1,6 ml de BSA 30%, 500 nM d'hydroxy-cholesterol (GE Healthcare, Piscataway, NJ) et 1 µg/ml d'insuline (SIGMA, St. Louis, MO). Toutes les cultures ont été incubées à 37°C dans une atmosphère humide avec 5% de CO₂.

Pour les dosages d'activité transcriptionnelle, des transfections transitoires des cellules LMH ont été réalisées en triple dans des plaques six puits à l'aide du protocole de lipofection FuGENE (Roche, Neuilly sur Seine, France). Dans chaque puits, les cellules ont été transfectées par 800 ng du plasmide rapporteur pGL2-BCM01-Luc et 20 ng du plasmide contrôle pCMV-bgal qui contient le gène bactérien β-galactosidase. Les cellules ont été rincées avec du PBS et incubées avec du milieu frais 24h après la transfection. Après 48h de transfection les cellules ont été lysées (lysis buffer - Promega, Madison, WI) et stockées à -80°C. Après 5 minutes de centrifugation à 12000 x g, les surnageants (10µl) ont été incubés 5s avec 40 µl du tampon de réaction luciférase (Promega, Madison, WI) incluant la luciférine (470 µM). L'activité de la luciférase est mesurée par un luminomètre (Veritas Turner Biosystems, Sunnyvale, XA). L'activité de la β-galactosidase est mesurée par hydrolysation de l'Ortho Nitro Phenyl Galactopyranoside (ONPG, SIGMA, St. Louis, MO) et utilisée pour normaliser l'activité de la luciférase.

### Résultats :

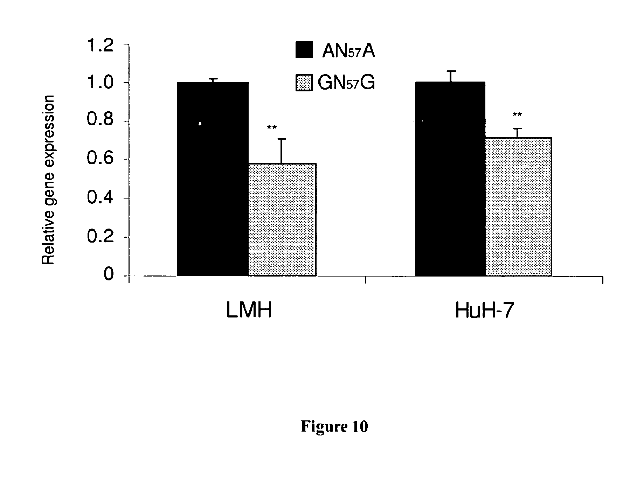

Dans les deux modèles cellulaires, la construction portant l'haplotype AN₅₇A est associée à une plus forte activité de luciférase que la construction GN₅₇G (P < 0.01) (**Fig. 10**). Ce résultat démontre clairement que l'haplotype GN₅₇G réduit l'activité du promoteur de *BCM01* et en conséquence le niveau d'ARNm de ce gène.

### EXEMPLE 5 : Caractérisation du niveau d'expression de BCMO1 dans le muscle Pectoralis Major d'animaux homozygotes AN₅₇A et GN₅₇G de la lignée à croissance rapide

L'objectif de cette étude était de valider, au sein d'une même lignée, l'effet des mutations sur le niveau d'expression de *BCMO1.*

### Dispositifs et prélèvement des échantillons

Les animaux étudiés appartenaient à la lignée expérimentale CR et étaient issus du croisement entre des parents hétérozygotes aux 2 mutations. L'étude a porté sur un total de 7 descendants femelles homozygotes AN₅₇A et 7 autres femelles homozygotes GN₅₇G. Ces animaux ont reçu un aliment standard distribué *ad libitum* jusqu'à l'abattage à 9 semaines. Pour chaque individu, un échantillon de muscle Pectoralis Major a été prélevé après la mort de l'animal, congelé immédiatement dans l'azote liquide et conservé à -80°C.

### Préparation des ARN, Transcription inverse et RT-PCR quantitative

La préparation des ARN, la transcription inverse et la détermination des niveaux d'expression des ARNm par RT-PCR quantitative sont effectuées comme décrit précédemment au point 2. de l'Exemple 2.

### Résultats

Comme attendu, l'expression de l'ARNr 18S n'est pas affectée par le génotype (Tableau 7). Cependant, les niveaux relatifs d'ARNm de *BCMO1* par rapport à l'ARNr 18S sont supérieurs d'un facteur 2.8 chez les animaux homozygotes AN₅₇A comparé à ceux observés chez les homozygotes GN₅₇G (p<0.001). Cette augmentation du niveau d'ARNm de *BCMO1* est associée à une diminution de la couleur jaune (b*) du muscle P. major (p<0.001) chez les animaux homozygotes AN₅₇A par rapport aux homozygotes GN₅₇G. Au sein de ce dispositif, la différence entre les femelles homozygotes AN₅₇A et GN₅₇G n'est pas significative pour la coloration rouge de la viande (a*= -1.2 et a*=-1.5, respectivement ; P=0.6).

**Tableau 7 : Niveaux d'expression en ARN dans le muscle pour 18S et BCMO1 (niveau absolu) en fonction du génotype (n=7 par génotype) et couleur jaune (b*) de la viande.**

| **Génotype** | **ARNr 18S** | **ARNm BCMO-I** | **BCMOI/18S** | **b*** |
|---|---|---|---|---|
| homozygote AN₅₇A | 0.84 ± 0.25 | 0.097 ± 0.017 | 0.123 ± 0.037 | 8.51 ± 1.03 |
| Homozygote GN₅₇G | 0.95 ± 0.18 | 0.039 ± 0.010 | 0.043 ± 0.018 | 10.99 ± 1.10 |
| Effet Génotype | 0.39 | <0.001 | <0.001 | <0.001 |

Cette étude confirme donc sur un second fond génétique (la lignée à croissance rapide) l'effet significatif des 2 mutations sur le niveau d'expression de *BCMO1* et la coloration jaune de la viande du filet.

### Conclusion : Via leur effet sur le niveau d'expression du gène BCMO1, les deux mutations liées dans le promoteur modulent la coloration jaune de la viande du filet de poulet.

### Bibliographie

○ Apple J.K., Roberts W.J., Maxwell C.V., Rakes L.K., Friesen K.G., Fakler T.M. (2007). Meat Science, 75, 640-647.
○ E. Baéza, M.R. Salichon, G. Marché, H. Juin (1998a) : Effect of sex on growth, technological and organoleptic characteristics of the Muscovy duck breast muscle. British Poultry Science 39, 398 - 403.
○ E. Baéza, G. Guy, M.R. Salichon, H. Juin, D. Rousselot-Pailley, D. Klosowska, G. Elminowska-Wenda, M. Srutek, A. Rosinski (1998b) : Influence of feeding systems, intensive vs extensive, on fatty liver and meat production in geese. Archiv für Geflügelkunde 62 (4), 169-175.
○ E. Baéza, M.R. Salichon, G. Marche, N. Wacrenier, B. Dominguez, J. Culioli (2000) : Effects of age and sex on the structural, chemical and technological characteristics of mule duck meat. British Poultry Science 41, 300-307.
○ P. Chartrin, K. Meteau, H. Juin, M.D. Bernadet, G. Guy, C. Larzul, H. Rémignon, J. Mourot, M.J. Duclos, E. Baéza (2006) : Effects of intramuscular levels on sensory characteristics of duck breast meat. Poultry Science, 85, 914-922.
○ X. Femandez, V. Santé, E. Baéza, E. Lebihan-Duval, C. Berri, H. Rémignon, R. Babilé, G. Le Pottier, T. Astruc (2002) : Effects of the rate of muscle post-mortem pH fall on the technological quality of turkey meat. British Poultry Science 43, 245-252.
○ Lebret B., Meusnier-Salaun M.C., Foury A., Mormède P., Dransfield E., Dourmad J.Y. (2007). Influence of rearing conditions on performance, behavioral and physiological responses of pigs to preslaughter handling, carcass traits and meat quality. Journal of Animal Science, 84 (9), 2436-2447.
○ Moloney A.P., Keane M.G., Dunne P.G., Mooney M.T., Troy D.J. (2008). Effect of concentrate feeding pattern in a grass silage/concentrate beef finishing system on performance, selected carcass and meat quality characteristics. Meat Science, 79 (2), 355-364.
○ NADAF JAVAD ET AL: "Identification of QTL controlling meat quality traits in an F-2 cross between two chicken lines selected for either low or high growth rate" BMC GENOMICS, vol. 8, juin 2007 (2007-06), page Article No.: 155,
○ Seyfert M., Mancini R.A., Hunt M.C., Tang J., Faustman C., Garcia M. (2006). Color stability, reducing activity and cytochrome C oxidase activity of five bovine muscles. Journal of Agriculture and Food Chemistry, 54 (23), 8919-8925.
○ Skrlep M., Candek-Potokar M. (2007). Pork color measurement as affected by bloom time and measurement location. Journal of Muscle Foods, 18 (1), 78-87.
○ Vestegaard M., Madsen N.T., Bligaard H.B., Bredhal L., Rasmussen P.T., Andersen H.R. (2007). Consequences of two or four months of finishing feeding of culled dry dairy cows on carcass characteristics and technological and sensory meat quality. Meat Science, 76 (4), 635-643.
○ KWOH et al., 1989, Proc Natl. Acad. Sci USA vol. 86 p : 1173-1177, 1989
○ LIZARDI et al. Biol. Technology, Vol.6, p :1197, 1988
○ Cole et al. In monoclonal antibodies and cancer therapy, Alan R. Liss, Inc., p77-96, 1985
○ Current protocols in Immunology, Coligan et al. ed., John Wiley & Sons, New York, 1994
○ (BRIGHMAN et al. Am. J. Med. Sci., vol. 298, p : 278-81, 1989
○ Nadaf et al., BMC Genomics 2007, 8 :155
○ Sibut et al., 2008. Journal of Animal Science, Jul 3. [Epub ahead of print]
○ Berri et al., 2001. Poultry Science, 80 : 833-838.
○ Le Bihan-Duval et al., 2008. BMC Genetics, 9 : 53
○ Gigaud et al., 2007. Septièmes Journées de la Recherche Avicole, Tours, 28-29 Mars 2007, 480-484.

## Revendications

1. Procédé pour différencier dans une population les animaux en fonction de la coloration de leur viande, **caractérisé en ce qu'**il comprend, pour un animal de ladite population les étapes suivantes :
a. Identification d'au moins une mutation associée à la sous-expression ou à la surexpression du gène BCMO1 (codant pour la beta-carotène monooxygénase 1) dans un échantillon biologique dudit animal ; et/ou
b. Analyse de l'expression du gène BCMO1 dans un échantillon biologique dudit animal ;
**caractérisé en ce que** lesdits animaux sont des volailles.

2. Procédé selon la revendication 1, caractérisé en que c'est un procédé de sélection d'animaux présentant une viande la plus colorée dans une population, **caractérisé en ce qu'**il comprend après l'étape b. une étape supplémentaire c. :
c. Sélection des animaux dont l'échantillon biologique présente une mutation associée à la sous-expression du gène BCMO1 et/ou une mutation associée à la diminution de l'activité de la protéine BCMO1, et/ou une sous-expression du gène BCMO1.

3. Procédé selon la revendication 1 ou 2, **caractérisé en ce que** la au moins une mutation est un SNP.

4. Procédé selon l'une des revendications 1 à 3, **caractérisé en ce que** la au moins une mutation est située dans la région promotrice du gène BCMO1.

5. Procédé selon l'une quelconque des revendications 1 à 4, **caractérisé en ce que** la au moins une mutation est la mutation A/G à la position 139 de SEQ ID N°2 et/ou la mutation A/G à la position 197 de SEQ ID N°2.

6. Procédé selon l'une quelconque des revendications 1 à 5, **caractérisé en ce que** l'animal est le poulet.

7. Procédé selon l'une quelconque des revendications 1 à 6, **caractérisé en ce que** le procédé comprend une étape préalable à l'étape a. de transformation des cellules dudit animal pour qu'elles présentent une mutation associée à la sous-expression du gène BCMO1 et/ou une sous-expression du gène BCMO1.

8. Procédé selon l'une quelconque des revendications 1 à 7, **caractérisé en ce que** l'expression du gène BCMO1 est analysée par la détection de l'expression des transcrits du gène BCMO1.

9. Procédé selon l'une quelconque des revendications 1 à 8, **caractérisé en ce qu'**il comprend une étape supplémentaire b'), après l'étape b), de comparaison du niveau d'expression du gène BCMO1 de l'échantillon biologique avec le niveau d'expression de référence du gène BCMO1 d'un témoin.

10. Utilisation d'un composé qui inhibe spécifiquement l'expression du gène BCMO1 pour l'obtention d'animaux présentant une viande plus colorée **caractérisé en ce que** ledit composé est un oligonucléotide, sélectionné dans le groupe comprenant des molécules d'ADN et d'ARN anti-sens, des ribozymes, des SiRNA et des aptamères.

11. Utilisation selon la revendication 10, **caractérisé en ce que** ledit oligonucléotide est un SiRNA sélectionné dans le groupe comprenant SEQ ID N°3 et 4.

## Patentansprüche

1. Verfahren zur Differenzierung von Tieren in einer Population je nach Farbe des Fleisches, **dadurch gekennzeichnet, dass** es für ein Tier der Population die folgenden Schritte umfasst:
(a) Identifizieren von mindestens einer Mutation, welche mit der Unterexpression oder Überexpression des Gens BCMO1 (kodierend für die Betacarotin Monooxygenase 1) in einer biologischen Probe des Tieres assoziiert ist; und/oder
(b) Analysieren der Expression des Gens BCMO1 in einer biologischen Probe des Tieres;
**dadurch gekennzeichnet, dass** die Tiere Geflügel sind.

2. Verfahren nach Anspruch 1, **dadurch gekennzeichnet, dass** das Verfahren ein Verfahren zur Selektion der Tiere ist, welche ein Fleisch aufweisen, welches das Farbigste in einer Population ist, **dadurch gekennzeichnet, dass** es nach dem Schritt (b) einen zusätzlichen Schritt (c) umfasst:
(c) Selektieren der Tiere, deren biologische Probe eine Mutation aufweist, welche mit der Unterexpression des Gens BCMO1 assoziiert ist, und/oder eine Mutation aufweist, welche mit der Aktivitätsabnahme des Proteins BCMO1, und/oder einer Unterexpression des Gens BCMO1 assoziiert ist.

3. Verfahren nach Anspruch 1 oder 2, **dadurch gekennzeichnet, dass** die mindestens eine Mutation ein SNP ist.

4. Verfahren nach einem der Ansprüche 1 bis 3, **dadurch gekennzeichnet, dass** die mindestens eine Mutation sich in der Promotorregion des Gens BCMO1 befindet.

5. Verfahren nach einem der Ansprüche 1 bis 4, **dadurch gekennzeichnet, dass** die mindestens eine Mutation die Mutation A/G in der Position 139 der SEQ ID Nr. 2 und/oder die Mutation A/G in der Position 197 der SEQ ID Nr. 2 ist.

6. Verfahren nach einem der Ansprüche 1 bis 5, **dadurch gekennzeichnet, dass** das Tier ein Huhn ist.

7. Verfahren nach einem der Ansprüche 1 bis 6, **dadurch gekennzeichnet, dass** das Verfahren vor dem Schritt (a) einen Transformationsschritt der Zellen des Tieres umfasst, damit sie eine Mutation aufweisen, welche mit der Unterexpression des Gens BCMO1 und/oder einer Unterexpression des Gens BCMO1 assoziiert sind.

8. Verfahren nach einem der Ansprüche 1 bis 7, **dadurch gekennzeichnet, dass** die Expression des Gens BCMO1 durch die Detektion der Expression der Transkripte des Gens BCMO1 analysiert wird.

9. Verfahren nach einem der Ansprüche 1 bis 8, **dadurch gekennzeichnet, dass** es einen zusätzlichen Schritt (b') nach dem Schritt (b) umfasst, um das Expressionsniveau des Gens BCMO1 der biologischen Probe mit dem Referenzexpressionsniveau des Gens BCMO1 einer Kontrollprobe zu vergleichen.

10. Verwendung einer Substanz, welche die Expression des Gens BCMO1 spezifisch hemmt, um Tiere zu erhalten, welche ein farbigeres Fleisch aufweisen, **dadurch gekennzeichnet, dass** die Substanz ein Oligonukleotid ist, welches aus der Gruppe umfassend Antisense DNA- und RNA-Moleküle, Ribozyme, siRNAs und Aptamere, ausgewählt wird.

11. Verwendung nach Anspruch 10, **dadurch gekennzeichnet, dass** das Oligonukleotid eine siRNA ist, welches aus der Gruppe umfassend SEQ ID Nr. 3 und 4, ausgewählt wird.

## Claims

1. Method for differentiating the animals in a population according to the colour of their meat, **characterized in that** it comprises, for an animal of said population, the following steps:
a. identifying at least one mutation associated with the underexpression or with the over-expression of the BCMO1 gene (encoding beta-carotene monooxygenase 1) in a biological sample from said animal; and/or
b. analysing the expression of the BCMO1 gene in a biological sample from said animal;
**characterized in that** said animals are poultry.

2. Method according to Claim 1, **characterized in that** it is a method for selecting animals having the most coloured meat in a population, **characterized in that** it comprises, after step b., an additional step c.:
c. selecting the animals from which the biological sample exhibits a mutation associated with the underexpression of the BCMO1 gene and/or a mutation associated with a decrease in the activity of the BCMO1 protein, and/or an underexpression of the BCMO1 gene.

3. Method according to Claim 1 or 2, **characterized in that** the at least one mutation is an SNP.

4. Method according to one of Claims 1 to 3, **characterized in that** the at least one mutation is located in the promoter region of the BCMO1 gene.

5. Method according to any one of Claims 1 to 4, **characterized in that** the at least one mutation is the A/G mutation at position 139 of SEQ ID No. 2 and/or the A/G mutation at position 197 of SEQ ID No. 2.

6. Method according to any one of Claims 1 to 5, **characterized in that** the animal is the chicken.

7. Method according to any one of Claims 1 to 6, **characterized in that** the method comprises a step, prior to step a., of transforming the cells of said animal so that they exhibit a mutation associated with the underexpression of the BCMO1 gene and/or an underexpression of the BCMO1 gene.

8. Method according to any one of Claims 1 to 7, **characterized in that** the expression of the BCMO1 gene is analysed by detecting the expression of the transcripts of the BCMO1 gene.

9. Method according to any one of Claims 1 to 8, **characterized in that** it comprises an additional step b'), after step b), of comparing the expression level of the BCMO1 gene of the biological sample with the reference expression level of the BCMO1 gene of a control.

10. Use of a compound which specifically inhibits the expression of the BCMO1 gene, for obtaining animals having a more coloured meat, **characterized in that** said compound is an oligonucleotide, selected from the group comprising antisense DNA and RNA molecules, ribozymes, SiRNAs and aptamers.

11. Use according to Claim 10, **characterized in that** said oligonucleotide is an SiRNA selected from the group comprising SEQ ID Nos. 3 and 4.


### SEQUENCE LISTING

<110> INRA
   LE BIHAN-DUVAL, Elisabeth
<120> MARQUEURS GENETIQUES POUR LA COLORATION DE LA VIANDE
<130> BCT 090236
<150> EP08305500.4 <151> 2008-08-25
<160> 39
<170> PatentIn version 3.3
<210> 1
   <211> 18518
   <212> DNA
   <213> gallus gallus
<220>
   <221> misc_feature
   <222> (11596)..(11605)
   <223> n is a, c, g, or t
<220>
   <221> misc_feature
   <222> (11678)..(11678)
   <223> n is a, c, g, or t
<220>
   <221> misc_feature
   <222> (13153)..(13162)
   <223> n is a, c, g, or t
<220>
   <221> misc_feature
   <222> (17489)..(17498)
   <223> n is a, c, g, or t
<400> 1
   catccttcta tgtaacagga aagagctgtt cttagcccag agaggagggg accgctacgc      60
   ctgcaggagc agctgggtag aggacacagg agagcgatgg agacaatatt taacagaaac     120
   aaagaagagc atccagagcc cataaaagct gaggtgcaag gtaagcggat gacaatctct     180
   tctaatactt gcagttactt tggtgttttc aaatatatac aacactgaaa cacttgctca     240
   ctcaaagcaa cgcagaaaag ggaaacagaa agtgagagac gtcagtgagt tttgtttaaa     300
   ggactgagct cagaccttat ttgtgccccc gactgtggga tttttctcat tttatgggca     360
   tttctatgtg ctttttcaga aagtccgtcc aaacaggatt cactgaagtt ggtggtgagg     420
   gacaaaatct caggatttaa tctggtggca ctccctcccc catggctccg tacagacttt     480
   cgttttctcc tctctgtaac ccttcactac cctctctagg gtagaaggta ttctagctaa     540
   aaatgcagag tatactccag tgagatagcc tgaagtggct ttctagaggt gatcaattta 600
   accttttttc tccttttttt tcaccttgaa agaaagggac acaaagagat ttttgctaca 660
   gctacacgag aggtttgttg ttgagtacct ctttctagta aatatacaca cagcaggcat 720
   ctcacaccag ttttaaagtc agttcccagg gaggtcatat caggtacata attacatgac 780
   tgtgttgttg ttgttcagtt tggtggctga attggaaaag attttgtttc aatgtgcttt 840
   gttttaattg gaggtgattt gatgcctttc aaatgtttgt cactcaaaat aaaagcttac 900
   tattgaaata tatgaatttg tttgcaagca acaactttta ctacagctta tctgtattat 960
   ggaatggttt atttgaacaa aaactgaaca gttgagtaaa ttaaattaac atattacttc 1020
   actggtcatg gtcattcagt atttaccagt aatgaaaatt aaagaggacc ctgacctctt 1080
   cacaaattta ccagtccctg agttctgcct gtattttgtc tccttgttac tcaatacgtg 1140
   cactgtaaat aggaccccat aacatcaacg tcaggcataa cttaactctt gctgtcctaa 1200
   agactgtaca gccacacgaa catccgatcc tccacggctg tgacagttaa tactctccca 1260
   aatatttgca gattttgctg tgccattttt gtgtcctctc atgtagtaca gaaaggcatt 1320
   gttattttca ctttagataa aagcacgagg caaaaaagag aagcaggcca ggtttgtaaa 1380
   gctatttatc acttaagaat gctgactgat acctactgtg atttgcagac gttcttaatt 1440
   cccacttaaa tgaactttca tctgcaaacc tcagtagatg tccattttct ctatgactta 1500
   aatattgcaa ttgtgccaga aattacactg aattacccca gaacaatcag aagctgtgtc 1560
   aagtctacag tgctttcaac aaaagactac ctttcttttt tcctgattat ccttgtcctg 1620
   gaattccttg agcatctttc ttctcatata gggaatatat ctctctatta gacatctata 1680
   taaagagatg tatgcctatc tatagataga tagatagaca atattaagag acatctaaat 1740
   atatagatga atttttttta actttgagac ttcaggtgat gtcgtgaaga ccagtacaag 1800
   tcacatatcc tatgaaatat gtttatttct gagaagctcg agcttttcct ggcatgagta 1860
   aaggggatta aacatatccc tttaaaaact gagtgcaagg aagaaagggc ccaatcttat 1920
   ttacttcctg gctttaagca atttcctgca gtcttttgct caagtgataa cgtcatatca 1980
   cagcatttat gcagaatatt acaagcaaca tttttgagca ggttatgctt tagttatccc 2040
   aaaccaaaac ataattactt gctattataa atatggactt gcattcacaa cccatctgcc 2100
   tatgaagcct atgcccttag taaaattgtc atgcggcatg ccccttccct aaaggaatga 2160
   atactggaat gctccttttg ttctcaggtc agttgcccac ttggttgcaa ggggtacttc 2220
   tccgaaatgg cccagggatg cacacaatag gggacactaa atacaaccac tggtttgatg 2280
   gcttggctct gctgcacagc ttcacgttta aaaatggtaa gttattgcat gtcagatcac 2340
   agaaagataa acaaaccaaa cattggtgtc ttatgttccc aattcagtgg ggcagtcctc 2400
   tctctggaac aattacatat tctaagacca acttcatgaa gtcatgcaaa caagaccaca 2460
   gttctttgtg ataactgagg gtaaaagttg tttcagccgc tcagtgtgag caagggccaa 2520
   tttctgcccc ttgtctgcag aatggagcgt gtgaagtgat aacacactta cggtaaagcg 2580
   tctccaaaca tacactcaca cctcactctc catagcagca gcagcagagg tatctctcca 2640
   ggcctgttct ccaaggcaga ttatattgtc ctttctgaag tgccaaagtt cagcgctgtc 2700
   tgcgctgtgg gatggagtgc agccagctct gagcaggtca ctgcagggca gcatttgctt 2760
   tccacacaca ccaagagggc catttttcat gaacactgaa gtgaaagcac tttctgatgc 2820
   ttcaggtact tttcagtgtg aggcaggatg ttttacgcct attcttccca taaaaagtac 2880
   agttctgtaa aagaactgag atcacccctg ttgttctcca ttcctggtgt ttacctttta 2940
   gtttccattt ctttaactgg aggcatttgg tgcagctgca gggtctccta gcctgtccag 3000
   gtctacatcc ccagcaaaat aacacataaa tcaatgagca gtgtttttta aattgctagt 3060
   tgttttactt ccctgacgca aactgtgctc agctagctca agtatatcag tccattagat 3120
   cagttagtgc agaagttggt tccttcaagc agtgtgttac tctgtcataa acagcgaagc 3180
   ctaagttctg ccaatactag agatttccta gtatccacct cccacacatt gctgcatcat 3240
   taatcatttt tacgctgatt cctttggttt actggaagaa tttatccaaa tccgttcttt 3300
   cctcacccta gtcccccgtg ttgtccaaac ttgatggtaa ttaaggacgg taatgaaatg 3360
   gttgctgatc tcagcagcca cgggaacagt cctgggttac tctatctttg tgccagtttt 3420
   ctaagaaacg tagtgtgaaa tgtgattgaa gtttgtttga cctttgtcag gtgaagttta 3480
   ctacagaagt aagtacctcc gaagtgacac atacaactgc aatatagaag caaaccgaat 3540
   cgtggtgtct gagtttggaa ccatggctta tccggatcca tgcaaaaaca tatttgccaa 3600
   gtaagcaatg cttttcccaa caggtcgctg ctatctacct ctctctccag cacggatgtc 3660
   gttctgttta ctcttggcac gtgaatacaa atatgaattt tgttttgtaa atttacaatt 3720
   gtcatctact gataaggtca ggagtgatac ccggtaccgt atgctctgtg ggttagtcta 3780
   aggcaaacat tttcattcca ctttgtcagt agtgtctgtt ttattcacta gtgcaaatag 3840
   attttgttgt agttttgaac aggcaagtac ttcctttttt cagaggtttc ctataatttt 3900
   ataaaaatga taccatctaa ggaataattg agagaaaaag caagaagctg ccagcaagac 3960
   tgcacctggg tgtccgactg tgcatagaaa tagcatttca tccatgcagg tccttgatcc 4020
   tcatgcaggc tctggctgtg aggaggtcct cctgcccctt gcaccctgcc tgctgaggca 4080
   gtgttggagt agtagagggg cactgcagga cagggacagg gccagcacag cctgctctgc 4140
   acagagccac ttgtgctttg cacggggtac ctgcaggtac agcttctgct cctctaatga 4200
   gccaatatga catttccctc ccctcagggc attctcatac ttatctcaca ccattcctga 4260
   gttcacggac aactgcctga tcaacattat gaaaactggg gatgattatt atgctaccag 4320
   tgagactaac ttcatcagaa aaattgatcc acagactctg gagacactag ataaggtata 4380
   aatgtctgtg tgtttcagac tgactcagat gaatccgttc aatctgcagt atacaaaatt 4440
   ggaaagactt gtatttcctc ccatttcctg gccagcagag tagacttttt tttgaaatcg 4500
   tggtaaaatg acctggtgag gatacttctt catgtaagaa gtcaggagtt caaaagaaaa 4560
   tcccaagcag tttagggaaa agtggggctt tgggcaattg cacaactgta cacggcgtag 4620
   tgaggttgca ggcataatac cacatatcgt gcttatgacg tggttatttt ggatgatttc 4680
   cattataatg catctgtagt tctcaggcga tttacataat taacagttta gtaggccatt 4740
   tatatgatga cttttcaagt gccttatgca agccattact taattgttaa aagtgtggac 4800
   atattgaatg ttgagttttt cttgtcatac actatggttt gagtacagtt cccttaaatt 4860
   gaatagaaat cattttgttc cagaaaattg ttagactcat tgccctgtta aataactgct 4920
   ctcagaatga aatgggcatt tgcagagtaa tgtctttagc caacctccct gctgtgttat 4980
   gtggctgcaa cggagaggtg aattcagatt ctgtagtttg cttagccttg aattaggaaa 5040
   tgatttgagt gcctgcatga gttatgtaac ttaaatgatg aattttcaag acctctgggg 5100
   ggacaccagt agaaggctac cagtgcagaa gggctggtgg atttgtggtg ttagctacga 5160
   tcaattttaa tgtacctacc catctctaaa ttctcctact gtatcatctt ccaggtagac 5220
   tacagcaaat atgtagctgt aaacttggca acttctcacc cacactatga cagtgctgga 5280
   aatattctca acatgggtac ttcaattgtt gataaaggga gaacaaaata tgttctcttt 5340
   aagatccctt cctctgtacc aggtaagata tggcttttat cagtatttct tagaaacttg 5400
   tcagcagttt cttttcttct gaggttaggg gaaaaacatc acaagatttt ataaatgcaa 5460
   ttttactttt tcttatttct gttcagaaaa agaaaagaag aaatcttgtt ttaaacacct 5520
   ggaagtagta tgctccatcc cttctcgctc cctgctccaa ccaagctact accacagctt 5580
   tggaatcaca gaaaattata ttgtgttcat agagcagcca tttaaactgg atattgtcaa 5640
   actggcaact gcctacatcc gaggtgtgaa ctgggcttcc tgcctttcct ttcataagga 5700
   ggataaggta ttctatccaa tgctgagcaa tggagtcagc caggccagtg acctgttgtt 5760
   actgttgctg ctctgttttt cgtcaagttc attaatttca gtaataaccc tttctttaaa 5820
   agcccttact gtcatgttgc actttataaa aattaatgtt gcatttttta aatattatct 5880
   cccatctcca gaaatttcac aaataaatgc tgatgctgga gtgacagttc agcatagcta 5940
   aaatgtgtgc cagaagagaa aataaaactg ctctagcatc ccagacatat acagctgctc 6000
   ctgttccata caaaatgcca tatagtgtgg atgaaatctg ttctagatcc atttcatcag 6060
   attaattaga gacaggttgg tggaaaagcc tgaatccaac aaaaggacag tctctttcat 6120
   tgaggcaatt aaggggaaaa aaaatctgtt tcctgagaac cttatttcca cattttgtgg 6180
   cccaagtggg gagagtgctg gagtagagaa cacccacaga gaagacagca gagaccctac 6240
   gctagggcag agttatttca gttaaaaata ggccagggga ttttccagat ttactggaaa 6300
   acttctacct tcagcttggc ccaaacagca atgtgcaaca gtgaaatgag tttggagcac 6360
   ttccacactc attttctact gcattttttt ggtctcaaga ttgaaacttc tcacctccta 6420
   gcagcaaatc ctgaatttgc ttcagtagaa ataggcagtg ctttagaatg tagatggcta 6480
   tctgtagaaa aatcatttcc tatgtaactg tttgctgatc attaatggct ttacataatg 6540
   tttgctctct ttcagacgtg gtttcacttt gtagacagaa agacgaaaaa agaagtatcc 6600
   accaagtttt acactgatgc tttggtgctt tatcaccaca taaatgctta cgaagaagat 6660
   ggccacgttg tttttgatat cgttgcctac agagacaata gcttgtatga tatgttttac 6720
   ttaaaaaaac tggacaaaga ctttgaagtg aacaacaagc ttacctccat cccaacctgc 6780
   aagcgctttg ttgtgcctct gcagtatgac aaggtaggaa gaaaagggct tttgaaactg 6840
   ctctttgatc agtaatgcga aagacatcaa tcagaaaagt gggtggatga gcagttgcca 6900
   gcccttttgt gcaaattttt ggttctttaa aaataacaga ttcacaaagt gttcaggagt 6960
   tttgtttttt gtatgtgttg tgagaaatgg caatgagcag ttggacgcac tgttacatgg 7020
   attgaggttc tgatgccatc atttatggag atttatggag ggttactgag catatatcat 7080
   ttccttagca gtgcaattat ttgtaagatg ctgtaaaata ttagcagata tatcaacttg 7140
   atgttagtta gaaaggtatt catgaggttt tgcacaatct gatttttcca ctttactgga 7200
   agcctatata aagtatattc acactcccca ctgttgcaat agctttatta aaatgagaag 7260
   atttcagcta agtgcaagat ttactagcaa caggaatgat tttatctttc aatataattt 7320
   aaaacatcat tttacatcat tgtttgcagc aaagattata tactggccag caacttgggc 7380
   aagttcctgc ttgattcatt tggaatggat catacttttt ccttctacat cccggaatct 7440
   tgctggcttt gcatgtctca aaaacttatg tagtaagggc aaaggatgtg ttggatctca 7500
   attgtcctac ctacgctgca atgttaagca ttcagctata gtatattcaa aacaagaatg 7560
   caccttaact ttggtgattg ttagtgtgaa tttcatgcct aatactttaa agcctatctc 7620
   cgttaattaa atgcacaaac tgtatcatct gtttattgtg cttctggaac acagccatat 7680
   ctctctaaac ctttctaaat gctcagtgtg attaaaagca gttacacaat gatacattca 7740
   agcccgctga gtataatgag acttctaagg agaaggtcag gatctaacac tacaggataa 7800
   atgaaggaac ttcatgaacc tggaatacct gtttaatccc aaatttgtct gttaatatca 7860
   aacctgatga aaattctgaa ttagaagcac attacatttc cagcgtacct ctctttcagg   7920
   atgcagaagt aggttctaat ttagtcaaac ttccaacttc cgcaactgct gtaaaagaaa   7980
   aagatggcag catctattgt caacctgaaa tattatgtga aggtaagaaa tatctgttat   8040
   acagatctca aatgcattct cttgtacacc tacacttaga tgtattctaa cagtgcgtag   8100
   cagctgttca tgtatgtcag atctttatta agctgaaagt aacaaaagct ttaattaata   8160
   tgttttgcat aatatggaca atatctttct cttccttatt tcactccttg gaatccatcc   8220
   attaatataa gcccctgaac ctgcacagga atgattcaga ggcagtattt agaagctgag   8280
   ttgttaagtt agggaataag tgaattgttg tcacttacaa tggtacccca gttctagaaa   8340
   catttatatt tagaaagttc aaaataccac tgtgtcaaag acagagagct tcactttgca   8400
   agtgttagat ctctgcgtgc ttactggaga cctgcacagc tttttgaaag gattacaaaa   8460
   gatctcctag gtcaggcaaa tcctcgttcc caaacagcat cccacagaaa ggtgagcatc   8520
   ctagcagtct ggggaatggg ttgctgtctc ctggatacat atttatcaag aatgaaatct   8580
   tttaaatgac tgcagttata tcagttttat attgctctag ggtcccaagc ctgatcttac   8640
   agctattaat gtggcttgca aatctacctg ttgtgaggta taaaatcgat aacttcagtt   8700
   atgtgctgta ttttatgaat ttcacctctg agagtggata tataaatttt atgaggggtc   8760
   ataacaggaa tttgtaaagg gtatggatgt gcttaaaggg agagtctttt cttggaatgt   8820
   taacttccag ttatatctca gaaactcaca tgtgatgtgt tgatgaaaca aatgaaatga   8880
   tacttataat ttccagtata atggattccc aagaatgaaa aattaccact tttgaacaaa   8940
   ctgccttggc ttaattgtgc tctctttgta cccactgtcg gctttttaga aatccaaaca   9000
   tataaaatct gttttaaatg ctgtcaattt tttttcatag ggtaacatta attagccgat   9060
   gtaaatgtat gcttagagtt atacttcttc ttttgttcct aagtcttggg ctttattata   9120
   cctagggata gaactgcctc gtgtcaacta tgactacaat ggcaaaaaat acaagtatgt   9180
   ctatgcaaca gaagtccagt ggagcccagt tcctacaaag gtatcactaa catttacaaa   9240
   gtcgggaact gtatatatat aacacaaatc aaatgtaagc agctataagt agaccatgct   9300
   tcagcctggc ccaaaataca ctgtcgtctg cagtcaccaa cccagattgg ggggttctga   9360
   gggcagagct gaaactgata aagcttctaa atgacaacac tgtgtattgt gttcataaat   9420
   ttcagcactt gaaagcaccg agattaaaag ctgggcatgt atgttagctt tcctgtataa   9480
   ttgaaacagc cccactgggc caaactattg aaaaaataca tcctcctttt gctagttgaa   9540
   ggaatggaac tttgttacca gctgttcaaa acatgtagct ccataaagag caaacatatg   9600
   ctttcttagg tgtgtgactt gagattggat gctgtaatat tcaactgaga atcggtttct   9660
   attaaaactt ttcccatctg tgagtcattc acatgcaaag caataggaaa tggctgaatt   9720
   ctcataaggg taagctcatg ttgtatcatt tcccttgtga agtattgata aagtcaggct 9780
   tctttatcca gtttttcatt tccagtcttc tgaatacaac agttacagag actgctgtat 9840
   ttccactcaa tttgattgaa cttctgtcat gtgccaaacc tacaaaggag agagtagcag 9900
   agaggaatag cattgggcac gcacatatct gtaataatta tgcttttttt tttcccttag 9960
   gaaactcaac taaaacaaga agttgttaaa ataatatgca atttttcagg aagctttatc 10020
   ctttggtaga gagctagttt tgtgtaacag gagagcaaag gcatcctttg tgtatgcgtg 10080
   atggcagctt ctcaggagac agcctagaat taccaactga ttaagatgaa aagtactgct 10140
   agtgaaggag tattgcacac acagactcac actttataat tactgtacgg agagcaaatt 10200
   gagatcactc ttggctctat ataattacca ccactgatta caaaataaag ccggatttct 10260
   tcacaacgca agaaaattta ttgcagaaca tttgacagta gctcaagcaa gtaagcacgt 10320
   ttgtgatttc cagtgcagat aatttctgcc tgacttaaaa tacgatctga cagcaattta 10380
   tatttatatt gctgcttatt cacatcagtt ttactttaat gaatgcagtc acggcaacaa 10440
   ccgtgtgcga tccttaggtc agtgaaactg ctgtcatctt ggatagaagg gaaaattctt 10500
   cctccagtgc ttgaatcact tgaaggggaa aagcataaaa ctgcatctgg atgtgtagaa 10560
   aaagcactta acagaactga ggtggaagcc tgtccccagt ggaaggtggt ttgggcagac 10620
   actgagccat taatgagaca aaaagctcga ccatgttcct ttggttaatg ccagtgtctg 10680
   tgtggaattc ttgccccatt ggtttaaccc ttacatctct attaatagcc cctgttctgg 10740
   catatatatc cagatatata tatccatatg tatccatata acaatagaag accttgaaga 10800
   atattgaggt cacaacaaaa ggaaaataat tcacaggatt agaaattgga aggttgaaat 10860
   cctgtttcct gctttagaac agaactgcag tcatttgggg caagtcactt caaagtgctg 10920
   tgccatgcac tcttctctgt atagggagga tgctattcac ccatgatgca gggtaagcaa 10980
   atcttaccac ctattagtat ttgtcaaata ttccaagccc aacgagggaa gatgtaatta 11040
   caatgtaatg aagtggtatc tatgcctttc ttaacactac taaagtagtc tctataaaat 11100
   taaagaacat cttacgatta tgcagctacc aaatgtgact cctaggttta acactttcca 11160
   ggcatttcct ttttgtgatg ccacttgcct cgttgttcct cacctctcag cactggtgtc 11220
   ccccacagca atctgttcat gttgcacctt tgggataaac cacttttctt ttctgatcac 11280
   aacttagatt gcaaaactga atgtccaaac aaaggaagta ctgcactggg gagaagacca 11340
   ctgctggccc tcagagccca tctttgttcc cagccccgat gcaagagaag aggatgaagg 11400
   taaaatagtg acaacatttt gaaagctggg ttttgcatta agtcttaaaa gtttcattgt 11460
   tgtcggtcag aacaaggaag ctaggtcgat gtttctgcag atcaattaaa attattgcca 11520
   accattttag tcctgcagaa taaagacaaa tcaatgtcag agcaaaagga gcttgcatga 11580
   agtcccatgc tattcnnnnn nnnnnagcat tcagctatag tatattcaaa acaagaatgc 11640
   accttaactt tggtgattgt tagtgtgaat ttcatgcnta atactttaaa gcctatctcc 11700
   gttaattaaa tgcacaaact gtatcatctg tttattgtgc ttctggaaca cagccatatc 11760
   tctctaaacc tttctaaatg ctcagtgtga ttaaaagcag ttacacaatg atacattcaa 11820
   gcccgctgag tataatgaga cttctaagga gaaggtcagg atctaacact acaggataaa 11880
   tgaaggaact tcatgaacct tgaatacctg tttaatccca aatttgtctg ttaatatcaa 11940
   acctgatgaa aattctgaat tagaagcaca ttacatttcc agcatacctc tctttcagga 12000
   tgcagaagta ggttctaatt tagtcaaact tccaacttcc gcaactgctg taaaagaaaa 12060
   agatggcagc atctattgtc aacctgaaat attatgtgaa ggtaagaaat atctgttata 12120
   cagatctcaa atgcattctc ttgtacacct acacttagat gtattctaac aatgcgtagc 12180
   agctgttcat gtatgtcaga tctttataaa gctgaaagta acaaaagctt taattaatat 12240
   gttttgcata atatggacaa tatctttctc ttccttattt cactccttgg agtccatcca 12300
   ttaatataag accctgaacc tgcacaggaa tgattcagag gcagtattta gaagctgagt 12360
   tgttaagtta gggaataagt gaattgttgt cacttacaat ggtaccccag ttctagaaac 12420
   atttatattt agaaagttca aaataccact gtgtcaaaga cagagagctt cactttgcaa 12480
   gtgttagatc tctgcgtgct cactggagac ctgcacagct ttttgaaagg attgcaaaag 12540
   atctcctagg tcaggcaaat cctggttccc aaacagcatc ccacagaaag gtgagcatcc 12600
   tagcagtctg gggaatgggt tgctgtctcc tggatacata tttatcaaga atgaaatctt 12660
   ttaaatgact gcagttatat cagttttata ttgctctagg gtcccaagcc tgatcttaca 12720
   gctattaatg tggcttgcaa atctacctgt tgtgaggtat aaaatcggta acttcagtta 12780
   tgtgctgtat tttatgaatt tcacctctga gagtggatat ttaaatttca tgaggggtca 12840
   taacaggaat ttgtaaaggg tatggatgtg cttaaaggga gagtcttttc ttggaatgtt 12900
   aatttccagt tatatctcag aaactcacat gtgatgtgtt gatgaaacaa atgaaatgat 12960
   acttataatt tccagtataa tggattccca agaatgaaaa attaccactt ttgaacaaac 13020
   tgccttggct taattgtgct ctctttgtac ccactgtcgg ctttttagaa atccaaacat 13080
   ataaaatctg ttttaaatgc tgtcaatttt ttttcatagg gtaacattaa ttagccgatg 13140
   taaatgtatg ctnnnnnnnn nnaaggttga aatcctgttt cctgctttag aacagaactg 13200
   cagtcatttg gggcaagtca cttcaaagtg ctgtgccatg cactcttctc tgtataggga 13260
   ggatgctatt cacccatgat gcagggtaag caaatcttac cacctattag tatttgtcaa 13320
   atattccaag cccaacgagg gaagatgtaa ttacaatgta atgaagtggt atctatgcct 13380
   ttcttaacac tactaaagta gtctctataa aattaaagaa catcttacga ttatgcagct 13440
   accaaatgtg actcctaggt ttaacacttt ccaggcattt cctttttgtg atgccacttg 13500
   cctcgttgtt cctcacctct cagcactggt gtcccccaca gcaatctgtt catgttgcac 13560
   ctttgggata aaccactgtc actgacactt ttcttttctg atcacaactt agattgcaaa 13620
   actgaatgtc caaacaaagg aagtactgca ctggggagaa gaccactgct ggccctcaga 13680
   gcccatcttt gttcccagcc ccgatgcaag agaagaggat gaaggtaaaa tagtgacaac 13740
   attttgaaag ctgggttttg cattaagtct taaaagtttc attgttgtcg gtcagaacaa 13800
   ggaagctagg tcgatgtttc tgcagatcaa ttaaaattat tgccaaccat tttagtcctg 13860
   cagaataaag acaaatcaat gtcagagcaa aaggagcttg catgaagccc catgctattc 13920
   ataaacagta aaatccatgc ttaggacttt ccagattgcc actgggaact agatttatca 13980
   gttgaagagg ctgaagctgt ggaattcagg gttgttacat atgagaacac aaagtggcat 14040
   tgcaagtctc cctgtaatct ggatccagtt ctgttctgag gcggctcctg gcaaaggcaa 14100
   atgacaaata gctgaaagga tgtgtcccta gcttcactac tcagttgccc aacaagactg 14160
   tcagggacac aactcagtgt ttgctttctg caggcaggcc aggtaaagga acagaagaga 14220
   accattcatt agttcttcag ctcccccagt cagacttcat ctagtaagca ctttcagttt 14280
   gtgtcctcag attcctagca aacaagactc atttcctaca gtgttatatt ttcagcttgg 14340
   tgtaaccagt atgagcttct aattctccat ttgtatgtca caggtgttgt tttgacctgt 14400
   gttgtggtgt ctgagccaaa taaagcaccc ttcctactca tcttggatgc taaaacattc 14460
   aaagaattgg gccgagccac agttaacgta gaaatgcatc tggacctgca tgggatgttt 14520
   ataccacaga atgatttggg ggctgagacg gaataaaacg ctattgatcc gactacacaa 14580
   actgagacaa ctttctactg aacatgagtt aatatccctt ttaccattca agaacaacca 14640
   tataacgaca caaaatgact atgtataatc tcttaaataa tagatataat ccttttaagg 14700
   cacagcgatg agttttacta caggtaacga tatgcacaac tggcatataa ctattccaaa 14760
   agaagaacga tcagtgtttt agaagtgcta atgttgtaca taacggcggc agagggaaca 14820
   ggagagaaag gtaacgggaa tatttaatag aatatagatt tctgagcaaa tgaagtgcag 14880
   tatttatggt gtgatgcatg gcatgagtca cataggtctg cagctcatgt atcttttaga 14940
   gatcgtttca agattgcagc ttgtgatgca agttttctcc agccagaaaa cctcatttta 15000
   aaccatctgc tactggtaat tcataccaat gcattttctt ggtgctcgat ttacactata 15060
   accaaagtta agtattacat tcaggtgcta caactttcta atttacaacc gaaacaaaca 15120
   agcaaacaac acttgctttg ctaataatcc catggtgtat ttttcctttt tatgatgaca 15180
   aaaccaagta catatggttt tatgtagcat tcaattatac ttcagtgcta ttccatccta 15240
   atgttataag caatttgtat ttaaatcagt tttccttgag aatatctgac ataacatttt 15300
   gtgtaatgag atgactatgt tgtctaaaga tgaacaggaa tgtatctttt attagtattg 15360
   ttaattgtgt tactaatact atgcatatga atgagagcaa tgtatttcta ggagaactca 15420
   gatatacatt caacaatttc tgtaggtgaa aatgcattta ctgatggaag ttgaatcgtt 15480
   aatgagggag aaaactgggt atccatccat ccaactatgt taggtgttca cctggtctgt 15540
   atgtgacacc acgctgtttg ggtatctctc actttcacat acctgttctc atggtttctg 15600
   ctactcactg tattttgcag gagagaaaca aaatgaaatc actgtcactt actatcgccc 15660
   catcacataa gaacaatggg gctttggtga cttgttcatg attacataag atgtttgcag 15720
   cggagcagca atagaaccaa caccatccac agttcttgct tgctctgtta tgactccctt 15780
   tgctgtcttt atggtttgca tgtatgaaga atacactgcc taattctaat gttaaaaagt 15840
   cactggggtc agatctagag cttaagtaag cagtttgggg ttttcaaatg tttatatatt 15900
   ccataaaatg gaaataaaca cctccataat atgtgtgtgc agaaatagtg atcatcagtg 15960
   ttctcaaggc atgttaccac cactctacag gagaattcta taatctaaag aagaaattga 16020
   attccatttg tgtacgtaac acagaagtag tactgagatg gcagactgac ctttggcgtt 16080
   gggaaaagta caaaggctgc tccaaaagta atgcctccta tattattctg ttggcccaca 16140
   atatcatagg cggatgttgg tggtacagca atagaagctg aaccttccca ccaatattcc 16200
   attccatttt gtcgtcgtgt gacagatggc agcagagggg cagcctgaca gaatggcatc 16260
   tgacatggaa gtgtgtacaa agcagagggg tgttactgaa ttcctctgtg cagaaaaaag 16320
   tggcacccac tgacattcat cgcttgccga acatttatgg agaccaaaca gtggaagtgg 16380
   gcacagtgag gcggtgggga cagcgacagt gggtcatctc ttctggtgca gatttttatg 16440
   agcgtggcat gcaggctctt gttcatcagt ggcagaaatg cagtgctaat ggtggtgact 16500
   gtgttgcaaa acagtgtttt gtggctgaga gtttgctcta tcaaatagtg tcattgtgct 16560
   ccgcgcatct cctgcagttt ccatggaaat aaataggagg cgatacttgc agactgaaca 16620
   aagtctccat gaatatttct ggaattataa gagggtcatc agtcaaaggt ccaaccatta 16680
   ttagagatct cattttaaga gttattctcc agaattccat ctgagatttc atagcgttgt 16740
   gtgtttggat gactgtgaat ttggatacag gattacagga tcctggggag ctaactcata 16800
   ccacagagaa gacataaaag ggaaatattc accataaaaa cagatcaggt tggttatcta 16860
   cagactaaat ctggatttgc taatctcagt atttgatcca ggtcatcccg caccacaggc 16920
   aatacagatc agagaccata cagctaatca caaatttctg agactatttt tagtggttgc 16980
   aatagtgatc tgatgaaaca tttaccacaa gtacagtggc atacggtata attttgaggc 17040
   tgacaatagc aagagggaat gcaggcgtgc tttgccaagt ggctgcaagg cttgtgggtg  17100
   ttttgttgtg gttgtacagc acatggtaga caagtccatc atttgctttg cttccctgac  17160
   atccttcacc atgaatgaga tcctgagggt cataagggcc ggatacttct tttgcaccag  17220
   tcccagtctt aagttagccc ctaaaactac gggggttcct gtgatggcag aaaaaaaccc  17280
   tacactcagt ggctgcaaat acaaatagta aagagagatc tctccctcat aggagaggaa  17340
   cagctgagcc acagaatcga gctgccggga tggacacatc tcatcacttc agagatgctg  17400
   tacgcagccc tctgagtggc acaggaggtt tggtgtgtag ctgtttttta tcagcgagaa  17460
   tgacatgcga atgaataacg aaatgtaann nnnnnnnnat aggtctgcag ctcatgtatc  17520
   ttttagagat cgtttcaaga ttgcagcttg tgatgcaagt tttctccagc cagaaaacct  17580
   cattttaaac catctgctac tggtaattca taccaatgca ttttcttggt gctcgattta  17640
   cactataacc aaagttaagt attacattca ggtgctacaa ctttctaatt tacaaccgaa  17700
   acaaacaagc aaacagcact tgctttgcta ataaccccat ggtgtatttt tcctttttat  17760
   gatgacaaaa ccaagtacat atggttttat gtagcattca attatacttc agtgctattc  17820
   catcctaatg ttataagcaa tttgtattta aatcagtttt ccttgagaat atctgacata  17880
   acattttgtg taatgagatg actatgttgt ctaaagatga acaggaatgt atcttttatt  17940
   agtattgtta attgtgttac taatactatg catatgaatg agagcaatgt atttctagga  18000
   gaactcagat atacattcaa caatttctgt aggtgaaaat gcatttactg atgaaagttg  18060
   aatcgttaat gagggagaaa actgggtatc catccatcca actatgttag gtgttcacct  18120
   ggtctgtatg tgacaccacg ctgtttgggt atctctcact ttcacatacc tgttctcatg  18180
   gtttctgcta ctcactgtat tttgcaggag agaaacaaaa tgaaatcact gtcacttact  18240
   atcgccccat cacataagaa caatggggct ttggtgactt gttcatgatt acataagatg  18300
   tttgcagcag agcagcaata gaaccaacac catccacagt tcttgcttgc tctgttatga  18360
   ctccctttgc tgtctttatg gtttgcatgt atgaagaata cactgcctaa ttctaatgtt  18420
   aaaaagtcac tggggtcaga tctagagctt aagtaagcag tctggggttt tcaaatgttt  18480
   atatgttcca taaaatggaa ataaacacct ccataata                          18518
<210> 2
   <211> 721
   <212> DNA
   <213> Gallus gallus
<400> 2
   ctctgcaatc acatgcttta tattgaaata attcttttcc tagaggattt taaaattaac    60
   aacattgaac tcagttaagt accttatctt gaaagaaatg catgaagtta aaaatccccc   120
   ttcttcttaa ttattcctrc catactttcg cagaggcttc gagttgctga taaccagrtg 180
   gaatcagatc acacgtrgga gaatgtagaa tagtgcctrt ttgtaaaaag gtgtttaatt 240
   caaaggtgtg ctgctcagtt cactctggaa ctgcttgtac ctaaagaaaa aaaagctcat 300
   cattgttcag tggtgatata ttgtgagtta atagaatagc ccaggttaaa tttgactgat 360
   gacactccat ctgtctataa cwcccaagaa gacaattcag aatggtgggt agagctttct 420
   cctgtttttc tccctgctcc tgccaagtgg grcaggcacc cctaccaaca gctccgactt 480
   tccctgcaat gctgaaggtg atatttcagt ttcattttgc ctttggtgtg cctggctgcc 540
   tgaactagct tcacacagag caaagaggtg agctatactt ttcttactct aacatcaaat 600
   tagtgaaaga gttaaaggga cgtgcgagaa ctactgtgta tgtgttaggg attaaagatc 660
   aactgttata agttgtcgat ctataagcca gcagccacta gctcaagttg cactttaacc 720
   t 721
<210> 3
   <211> 21
   <212> RNA
   <213> Artificial
<220>
   <223> SiRNA
<400> 3
   cuaucgcccc aucacauaag a 21
<210> 4
   <211> 21
   <212> RNA
   <213> Artificial
<220>
   <223> SiRNA
<400> 4
   uuaugugaug gggcgauagu a 21
<210> 5
   <211> 423
   <212> DNA
   <213> gallus gallus
<220>
   <221> variation
   <222> (112)..(113)
   <223> insertion of a T
<400> 5
   taccactttt gaacaaactg ccttggctta attgtgctct ctttgtaccc actgtcggct 60
   ttytagaaat ccaaacatat aaaatctgtt ttaaatgctg tcaatttttt ttcayagggt 120
   aacattaatt agcckatgta aatgtatgct tagagttata cttcttcttt tgttyctaag 180
   tcttgggctt tattatacct agggatagaa ctgcctcgtg tcaactatga ctacaatggc 240
   aaaaaataca agtatgtcta tgcaacagaa gtccagtgga gcccagttcc tacaaaggta 300
   tcactaacat ttacaaagtc gggaactgta tatatataac acaratcaaa tgtaagcagc 360
   tataagtaga ccatgcttca gcctggccca aaatacactg tcgtctgcag tcaccaaccc 420
   aga 423
<210> 6
   <211> 366
   <212> DNA
   <213> gallus gallus
<400> 6
   agattcctag caaacaagac tcatttccta cagtgttata ttttcagctt ggtgtaacca 60
   gtatgagctt ctaattctcc atttgtatgt cacaggtgtt gttttgacct gtgttgtggt 120
   gtctgagcca aataaagcac ccttcctact catcttggat gctaaaacat tcaaagaatt 180
   gggccgagcc acagttaacg tagaaatgca tctggacctg catgggatgt ttataccaca 240
   gaatgatttg ggggctraga cggartaaaa cgctattgat ccgactacac aaactgagac 300
   aactttctac tgaacatgag ttaatatccc ttttaccatt caagaacaac catataacga 360
   cacaaa 366
<210> 7
   <211> 25
   <212> DNA
   <213> Artificial
<220>
   <223> PCR primer
<400> 7
   acaatgtaat gaagtggtat ctatg 25
<210> 8
   <211> 18
   <212> DNA
   <213> Artificial
<220>
   <223> PCR primer
<400> 8
   tgcaagctcc ttttgctc 18
<210> 9
   <211> 21
   <212> DNA
   <213> Artificial
<220>
   <223> PCR primer
<400> 9
   agattcctag caaacaagac t 21
<210> 10
   <211> 23
   <212> DNA
   <213> Artificial
<220>
   <223> PCR primer
<400> 10
   tttgtgtcgt tatatggttg ttc 23
<210> 11
   <211> 24
   <212> DNA
   <213> Artificial
<220>
   <223> PCR primer
<400> 11
   aagatcaact gttataagtt gtcg 24
<210> 12
   <211> 25
   <212> DNA
   <213> Artificial
<220>
   <223> PCR primer
<400> 12
   ctgcaagtat tagaagagat tgtca 25
<210> 13
   <211> 21
   <212> DNA
   <213> Artificial
<220>
   <223> PCR primer
<400> 13
   tatgaagcct atgcccttag t 21
<210> 14
   <211> 22
   <212> DNA
   <213> Artificial
<220>
   <223> PCR primer
<400> 14
   tgaattggga acataagaca cc 22
<210> 15
   <211> 22
   <212> DNA
   <213> Artificial
<220>
   <223> PCR primer
<400> 15
   gaaacgtagt gtgaaatgtg at 22
<210> 16
   <211> 23
   <212> DNA
   <213> Artificial
<220>
   <223> PCR primer
<400> 16
   atatttgtat tcacgtgcca aga 23
<210> 17
   <211> 17
   <212> DNA
   <213> Artificial
<220>
   <223> PCR primer
<400> 17
   ctgcaggtac agcttct 17
<210> 18
   <211> 21
   <212> DNA
   <213> Artificial
<220>
   <223> PCR primer
<400> 18
   gccaggaaat gggaggaaat a 21
<210> 19
   <211> 17
   <212> DNA
   <213> Artificial
<220>
   <223> PCR primer
<400> 19
   atgggcattt gcagagt 17
<210> 20
   <211> 19
   <212> DNA
   <213> Artificial
<220>
   <223> PCR primer
<400> 20
   gagcagcaac agtaacaac 19
<210> 21
   <211> 23
   <212> DNA
   <213> Artificial
<220>
   <223> PCR primer
<400> 21
   atttcctatg taactgtttg ctg 23
<210> 22
   <211> 17
   <212> DNA
   <213> Artificial
<220>
   <223> PCR primer
<400> 22
   caactgctca tccaccc 17
<210> 23
   <211> 24
   <212> DNA
   <213> Artificial
<220>
   <223> PCR primer
<400> 23
   attagaagca cattacattt ccag 24
<210> 24
   <211> 23
   <212> DNA
   <213> Artificial
<220>
   <223> PCR primer
<400> 24
   acatctaagt gtaggtgtac aag 23
<210> 25
   <211> 22
   <212> DNA
   <213> Artificial
<220>
   <223> PCR primer
<400> 25
   taccactttt gaacaaactg cc 22
<210> 26
   <211> 20
   <212> DNA
   <213> Artificial
<220>
   <223> PCR primer
<400> 26
   tctgggttgg tgactgcaga 20
<210> 27
   <211> 21
   <212> DNA
   <213> Artificial
<220>
   <223> PCR primer
<400> 27
   ctctgcaatc acatgcttta t 21
<210> 28
   <211> 21
   <212> DNA
   <213> Artificial
<220>
   <223> PCR primer
<400> 28
   tctggatgct cttctttgtt t 21
<210> 29
   <211> 23
   <212> DNA
   <213> Artificial
<220>
   <223> PCR primer
<400> 29
   gaactcagtt aagtacctta tct 23
<210> 30
   <211> 24
   <212> DNA
   <213> Artificial
<220>
   <223> PCR primer
<400> 30
   agcagcacac ctttgaatta aaca 24
<210> 31
   <211> 25
   <212> DNA
   <213> Artificial
<220>
   <223> PCR primer
<400> 31
   gcgaaagtat ggcaggaata attaa 25
<210> 32
   <211> 22
   <212> DNA
   <213> Artificial
<220>
   <223> PCR primer
<400> 32
   aggcttcgag ttgctgataa cc 22
<210> 33
   <211> 32
   <212> DNA
   <213> Artificial
<220>
   <223> PCR primer
<400> 33
   ctattctaca ttctcccacg tgtgatctga tt 32
<210> 34
   <211> 23
   <212> DNA
   <213> Artificial
<220>
   <223> PCR primer
<400> 34
   aacaaagaag agcatccaga gcc 23
<210> 35
   <211> 20
   <212> DNA
   <213> Artificial
<220>
   <223> PCR primer
<400> 35
   gccaagccat caaaccagtg 20
<210> 36
   <211> 15
   <212> PRT
   <213> Artificial
<220>
   <223> synthetic peptide
<400> 36
   Met Glu Thr Ile Phe Asn Arg Asn Lys Glu Glu His Pro Glu Pro
   1               5                   10                  15
<210> 37
   <211> 16
   <212> PRT
   <213> Artificial
<220>
   <223> synthetic peptide
<400> 37
   Asp Lys Asp Phe Glu Val Asn Asn Lys Leu Thr Ser Ile Pro Thr Cys
   1               5                   10                  15
<210> 38
   <211> 19
   <212> DNA
   <213> artificial sequence
<220>
   <223> BCMO1F
<400> 38
   agcctgtgat ttcctctgc 19
<210> 39
   <211> 20
   <212> DNA
   <213> Artificial sequence
<220>
   <223> primer BCMO1R
<400> 39
   tcgctctcct gtgtcctcta 20